(19) **Europäisches Patentamt / European Patent Office / Office européen des brevets**

(11) **EP 1 495 727 B1**

(12) **EUROPEAN PATENT SPECIFICATION**

(45) Date of publication and mention
of the grant of the patent:
**24.12.2008 Bulletin 2008/52**

(51) Int Cl.:
***A61B 17/22*** *(2006.01)*

(21) Application number: **04015977.4**

(22) Date of filing: **07.07.2004**

(54) **Ultrasonic surgical system and probe**

Chirurgisches Ultraschallsystem und Probe

Système de chirurgie à ultrasons et sonde

(84) Designated Contracting States:
**DE FR GB**

(30) Priority: **07.07.2003 JP 2003271455**

(43) Date of publication of application:
**12.01.2005 Bulletin 2005/02**

(73) Proprietor: **Olympus Corporation
Tokyo 151-0072 (JP)**

(72) Inventor: **Ono, Hiroo, Intellectual Property
Department
Hachioji-shi
Tokyo 192-8512 (JP)**

(74) Representative: **von Hellfeld, Axel
Wuesthoff & Wuesthoff
Patent- und Rechtsanwälte
Schweigerstrasse 2
81541 München (DE)**

(56) References cited:
**US-A- 4 979 952     US-A- 5 026 387
US-A- 5 279 547     US-A- 5 425 704**

**Description**

BACKGROUND OF THE INVENTION

1) Field of the Invention

The present invention relates to an ultrasonic surgical system and a probe for performing surgical treatments such as coagulation and cutting of living tissues, lithotrity, and suctioning using ultrasonic vibration.

2) Description of the Related Art

As an ultrasonic surgical system for surgical operation using ultrasonic vibration, an ultrasonic coagulating and cutting apparatus which cuts or removes a living tissue using a probe which transmits the ultrasonic vibration, and an ultrasonic lithotrite which breaks a calculus in a hollow portion of a body and sucks in broken particles of the calculus have been developed. The ultrasonic vibration in such an ultrasonic surgical system is realized by controlling driving of an ultrasonic transducer incorporated into a handpiece. Normally, the ultrasonic transducer is desirably driven with its basic resonance frequency or a frequency near the basic resonance frequency. When a probe which transmits the ultrasonic vibration contacts with a surgical instrument such as a forceps or a rigid endoscope, a heavy mechanical load is exerted on the probe and an impedance of the probe is thereby increased. Therefore, when the probe contacts with the surgical instrument, it is necessary to prevent damage to the probe by stopping driving the ultrasonic transducer or by warning an operator.

Examples of prior art ultrasonic surgical systems are described in the granted United States Patent Nos. 4 979 952, 5 026 387, and 5 425 704.

US 4 979 952 discloses the use of a first reference parameter (impedance Z3 determined empirically) and of a second parameter (impedance) in order to decrease power when the probe is idling, i.e. when the impedance is lower than the first reference parameter. It also uses a parameter Q = f0 / $\Delta$f in order to detect if the probe is idling.

US 5 279 547 discloses a transducer, a probe a detector which detects a resonance frequency, a driver, storage unit and a controller. The load exerted on the probe is here increased when the probe contacts a harder tissue.

SUMMARY OF THE INVENTION

It is an object of the present invention to at least solve the problems in the conventional technology. The present invention provides an ultrasonic surgical system as defined in claim 1 and claim 7. Preferred features of the invention are recited in the dependent claims.

An ultrasonic surgical system according to one aspect of the present invention includes an ultrasonic transducer, a probe connected to the ultrasonic transducer and coming in contact with a treatment target, a detector detecting current and voltage which are supplied to the ultrasonic transducer, a driver driving the ultrasonic transducer to oscillate at its resonance point, and a controller. The controller detects a mechanical load exerted on the probe based on the voltage detected by the detector, and outputs a signal for reducing the mechanical load to the driver when the detected mechanical load is higher than a predetermined value.

An ultrasonic surgical system according to another aspect of the present invention includes an ultrasonic transducer, a probe connected to the ultrasonic transducer and coming in contact with a treatment target, a detector detecting a resonance frequency from a driving signal input to the ultrasonic transducer, a driver driving the ultrasonic transducer to oscillate at a resonance point of the ultrasonic transducer, and a storage unit storing a first reference parameter for determining whether a hardness of an object in contact with the probe is a hardness which causes damage to the probe. The ultrasonic surgical system also includes a controller outputting a signal for reducing a mechanical load exerted on the probe to the driver when the resonance frequency detected by the detector is higher than the first reference parameter.

The probe used in the ultrasonic surgical system according to the present invention, preferably includes an ultrasonic vibration transmitting portion which transmits an ultrasonic vibration supplied from an ultrasonic transducer, and a protecting member which detachably covers a surface of the ultrasonic vibration transmitting unit excluding a predetermined region from a distal end of the ultrasonic vibration transmitting portion.

The other objects, features, and advantages of the present invention are specifically set forth in or will become apparent from the following detailed description of the invention when read in conjunction with the accompanying drawings.

## BRIEF DESCRIPTION OF THE DRAWINGS

Fig. 1 is a schematic block diagram of an ultrasonic surgical system according to a first embodiment of the present invention;

Fig. 2 is a block diagram which depicts the basic configuration of a control device of the ultrasonic surgical system according to the first embodiment;

Fig. 3 is an example of a fluctuation in a set current set by an output control unit;

Fig. 4 is an example of the relationship between impedance and frequency of an ultrasonic transducer;

Fig. 5 is a flowchart of respective process procedures performed until a control unit controls driving of the ultrasonic transducer according to a result of an impedance comparing process;

Fig. 6 is an example of a fluctuation in a set current set by the output control unit if amplitude is modulated;

Fig. 7 is a flowchart of respective process procedures performed until the control unit controls driving of the ultrasonic transducer according to a result of impedance comparing process if the amplitude is modulated;

Fig. 8 is a block diagram which depicts the basic configuration of a control device of the ultrasonic surgical system according to a second embodiment;

Fig. 9 is an example of the relationship between driving power and frequency of the ultrasonic transducer;

Fig. 10 is a flowchart of process procedures performed until the control unit controls driving of the ultrasonic transducer according to a result of a driving power comparing process;

Fig. 11 is a flowchart of respective process procedures performed until the control unit controls driving of the ultrasonic transducer according to a result of a driving power comparing process if amplitude is modulated;

Fig. 12 is a block diagram which depicts the basic configuration of a control device of the ultrasonic surgical system according to a third embodiment;

Fig. 13 is a flowchart of respective process procedures performed until the control unit controls driving of the ultrasonic transducer according to a result of a driving power comparing process;

Fig. 14 is an example of a fluctuation in resonance frequency;

Fig. 15 is a flowchart of respective process procedures performed until the control unit controls the driving of the ultrasonic transducer according to a result of n comparing processes to the resonance frequency;

Fig. 16 is an example of a fluctuation in resonance frequency when a probe is in contact with a hard object;

Fig. 17 is an example of a fluctuation in resonance frequency when a probe is in contact with a soft object;

Fig. 18 is an example of a fluctuation in resonance frequency when the probe is in contact with a calculus;

Fig. 19 is a block diagram which depicts the basic configuration of a control device of the ultrasonic surgical system according to a fourth embodiment;

Fig. 20 is a schematic view which depicts an example of an arrangement state of wirings on a probe of an ultrasonic surgical system according to a fourth embodiment of the present invention;

Fig. 21 is an example of an electric connection state of wirings arranged on the probe of the ultrasonic surgical system according to the fourth embodiment of the present invention;

Fig. 22 is a schematic view which depicts an example of an arrangement state of a wiring on a probe of an ultrasonic surgical system according to a first modification of the fourth embodiment of the present invention;

Fig. 23 is a schematic view which depicts an example of an arrangement state of a wiring on a probe of an ultrasonic surgical system according to a second modification of the fourth embodiment of the present invention;

Fig. 24 is an example of an electric connection state of wirings arranged on a probe of an ultrasonic surgical system according to a second modification of the fourth embodiment of the present invention;

Fig. 25 is a schematic view which depicts an example of an arrangement state of wirings on a probe of an ultrasonic surgical system according to a third modification of the fourth embodiment of the present invention;

Fig. 26 is a schematic view which depicts an example of a protecting tool arranged on a probe of an ultrasonic surgical system according to a fifth embodiment of the present invention; and

Fig. 27 is a schematic view of the protecting tool partially arranged on the probe of ultrasonic surgical system according to the fifth embodiment of the present invention.

## DETAILED DESCRIPTION

Exemplary embodiments of an ultrasonic surgical system and a probe will be explained below in detail with reference to the accompanying drawings. As an ultrasonic surgical system of the present invention, exemplary embodiments of an ultrasonic lithotrite which breaks a calculus in a hollow portion of a body and sucks in broken particles of the calculus will be explained.

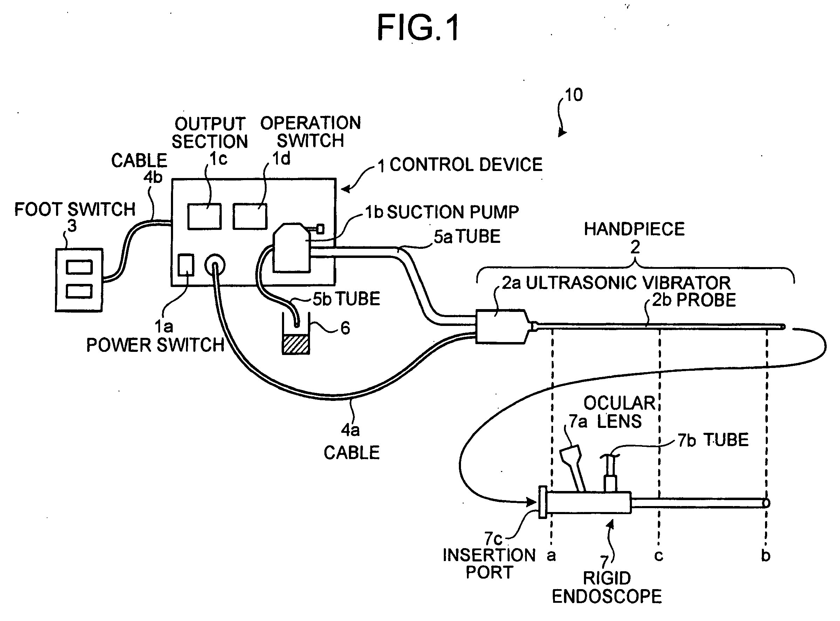

Fig. 1 is a schematic block diagram of the ultrasonic surgical system according to a first embodiment of the present invention. The ultrasonic surgical system 10 includes a control device 1, a handpiece 2, and a foot switch 3. The control device 1 includes a power switch 1a, a suction pump 1b, an output section 1c, and an operation switch 1d. The handpiece 2 includes an ultrasonic transducer 2a consisting of a piezoelectric ceramic or the like, and a probe 2b. The ultrasonic transducer 2a is electrically connected to the control device 1 through a cable 4a, and connected to the suction pump 1b through a tube 5a. The suction pump 1b includes a tube 5b communicating with the tube 5a. The foot switch 3 includes a pedal, and is electrically connected to the control device 1 through a cable 4b.

The probe 2b consists of, for example, titanium or titanium alloy, and is detachably connected to the ultrasonic transducer 2a. The probe 2b can be, for example, screwed into the ultrasonic transducer 2a or fitted into the ultrasonic transducer 2a using a spring. By connecting the probe 2b to the ultrasonic transducer 2a, an ultrasonic vibration output from the ultrasonic transducer 2a can be transmitted to the probe 2b. The ultrasonic transducer 2a and the probe 2b include a through hole (not shown) which ranges from a distal end to a connection portion between the ultrasonic transducer 2a and the tube 5a. When the suction pump 1b starts its suction operation, a treatment target such as a calculus near the distal end of the probe 2b is sucked toward the suction pump 1b through the through hole of the ultrasonic transducer 2a and the probe 2b and the tube 5a. The suction operation is not hindered by the connection between the ultrasonic transducer 2a and the probe 2b.

A rigid endoscope 7 includes an ocular lens 7a, a tube 7b in which a perfusion solution such as a physiological saline solution flows, and an insertion port 7c through which the probe 2b is inserted. The rigid endoscope 7 also includes therein a through hole (not shown) in a longitudinal direction. The probe 2b is inserted into the rigid endoscope 7 through the insertion port 7c. The inserted probe 2b can be detached from the rigid endoscope 7. A gap is present between the inserted probe 2b and the through hole of the rigid endoscope 7 to the extent that the probe 2b can be freely operated. The through hole introduces the perfusion solution flowing into the through hole from the tube 7b to neighborhoods of a distal end of the rigid endoscope 7. Each of the probe 2b and the rigid endoscope 7 is preferably made of a material which can resist a harsh sterilization treatment performed by an autoclave or the like.

In the control device 1, when the power switch 1a is turned on, a set value relating to an ultrasonic output (set ultrasonic output) is input through the operation switch 1d, and a driving command is input from the foot switch 3, the ultrasonic transducer 2a outputs an ultrasonic vibration corresponding to the set ultrasonic output, which ultrasonic vibration is propagated through the probe 2b. The enables the ultrasonic surgical system 10 to perform a desired medical treatment to the treatment target such as the calculus or a living tissue. If the calculus in the hollow portion of the body is to be broken and sucked in, for example, an operator brings the probe 2b accompanied by the ultrasonic vibration into contact with the calculus in the hollow portion of the body. The calculus in contact with the probe 2b is broken and sucked in, along with the perfusion solution supplied from the tube 7b, by the suction pump 1b. The suction treatment is carried out by making the distal end of the probe 2b closer to the broken particles of the calculus while the suction pump 1b is driven. The suction pump 1b includes the tube 5b, and discharges the sucked perfusion solution and calculus to a bottle 6. The set ultrasonic output input by the operator is a set value relating to the ultrasonic output of the ultrasonic transducer 2a such as a current, a voltage, a power, or a driving frequency at or with which the ultrasonic transducer 2a is driven.

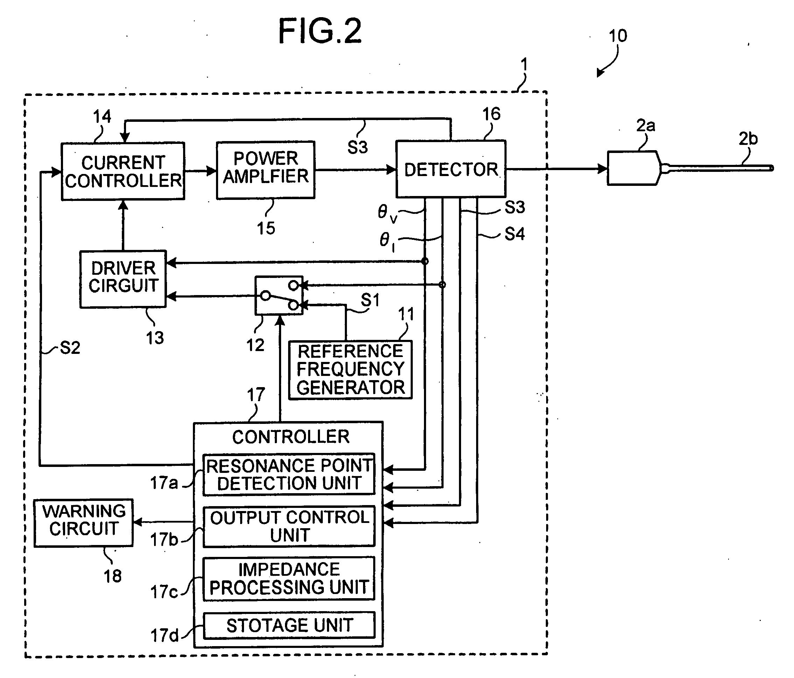

The control device 1 of the ultrasonic surgical system 10 will be explained in detail. Fig. 2 is a block diagram which depicts the basic configuration of the control device 1. With reference to Fig. 2, the control device 1 includes a reference frequency generator 11, a switch circuit 12, a driver circuit 13, a current controller 14, a power amplifier 15, a detector 16, a controller 17, and a warning circuit 18. The switch circuit 12, the driver circuit 13, the current controller 14, and power amplifier 15, and the controller 17 are connected to the detector 16. The driver circuit 13, the current controller 14, the power amplifier 15, and the detector 16 form one feedback loop, whereas the current controller 14, the power amplifier 15, and the detector 16 forms another feedback loop. The reference frequency generator 11, the driver circuit 13, and the detector 16 are connected to the switch circuit 12 so as to selectively supply a signal output from the reference frequency generator 11 or a signal fed back from the detector 16 to the driver circuit 13. The detector 16 is connected to the ultrasonic transducer 2a of the handpiece 2. The controller 17 controls the switch circuit 12, the current controller 14, and the warning circuit 18.

The reference frequency generator 11 is a generator which oscillates with a resonance frequency fr or a frequency near the resonance frequency fr as a reference frequency. The reference frequency generator 11 outputs a reference frequency signal S1 corresponding to this reference frequency to the switch circuit 12.

The switch circuit 12 functions to switch the signal supplied to the driver circuit 13 under control of the controller 17, and selectively supplies the reference frequency signal S1 or the signal fed back from the detector 16 to the driver circuit 13. The switch circuit 12 selects the reference frequency signal S1 when the ultrasonic transducer 2a is activated, and selects the signal fed back from the detector 16 when the controller 17 detects the resonance frequency fr.

The driver circuit 13 is an analog phase synchronization circuit, and composed of, for example, a phase comparator, a lowpass filter (LPF), and a voltage controlled oscillator (VCO). The driver circuit 13 oscillates with a driving frequency for driving the ultrasonic transducer 2a, and outputs a driving signal corresponding to the driving frequency. The driver circuit 13 may be a digital phase synchronization circuit composed of a phase comparator, a direct digital synthesizer (DDS), and an UP/DOWN counter.

The driver circuit 13 oscillates with the reference frequency corresponding to the reference frequency signal S1, and outputs the driving signal with the reference frequency as the driving frequency when the ultrasonic transducer 2a is activated and the reference frequency signal S1 is input to the driver circuit 13. When the ultrasonic transducer 2a is activated and the controller 17 detects the resonance frequency Fr, a voltage phase signal $\theta_V$ and a current phase signal $\theta_I$ fed back from the detector 16 are input to the driver circuit 13. The voltage phase signal $\theta_V$ and the current phase signal $\theta_I$ correspond to a voltage phase and a current phase of the driving signal input to the ultrasonic transducer 2a, respectively. The current phase signal $\theta_I$ is input to the driver circuit 13 through the switch circuit 12 as explained above. In this case, the driver circuit 13 detects a phase difference between a current and a voltage of the driving signal from the input voltage phase signal $\theta_V$ and current phase signal $\theta_I$, and oscillates with the frequency (resonance frequency fr) with which the phase difference is zero. The driver circuit 13 can thereby output the driving signal with the resonance frequency fr as the driving frequency. The driver circuit 13 then exercises a PLL control for controlling the phase difference between the current and the voltage of the driving signal to be zero based on the voltage phase signal $\theta_V$ and the current phase signal $\theta_I$ fed back from the detector 16. As a result, the driver circuit 13 keeps oscillating with the resonance frequency fr, and outputs the driving signal with the resonance frequency fr as the driving frequency.

The driving signal output from the driver circuit 13 is supplied to the current controller 14. The current controller 14 is composed of, for example, a differential amplifier circuit and a multiplier circuit. The current controller 14 determines a current amplification factor of the driving signal input from the driver circuit 13 based on a set current $|I|_{set}$ set by the controller 17 and a current $|I|$ of the driving signal detected by the detector 16, and makes the current of the driving signal closer to the set current $|I|_{set}$. Namely, the current controller 14 exercises a constant current control for setting the current $|I|$ of the driving signal input to the ultrasonic transducer 2a to be substantially equal to the set current $|I|_{set}$. In this constant current control, a current setting signal S2 corresponding to the set current $|I|_{set}$ is input from the controller 17 to the current controller 14. A current signal S3 corresponding to the current $|I|$ of the driving signal input to the ultrasonic transducer 2a is input from the detector 16 to the current controller 14. Amplitude of the ultrasonic vibration output from the ultrasonic transducer 2a is proportional to the current $|I|$ of the driving signal. Further, the current controller 14 exercises the constant current control for setting the current $|I|$ of the driving signal to be substantially equal to the set current $|I|_{set}$, whereby the ultrasonic transducer 2a can output the ultrasonic transducer at the amplitude corresponding to the set current $|I|_{set}$. The current controller 14 then outputs the driving signal at the current substantially equal to the set current $|I|_{set}$.

The driving signal output from the current controller 14 is input to the power amplifier 15. The power amplifier 15 is composed of a well-known amplifier circuit which amplifies a power of an input signal, and amplifies the power of the input driving signal. The amplified driving signal is input to the ultrasonic transducer 2a. The ultrasonic transducer 2a can thereby transmit the ultrasonic vibration corresponding to the set ultrasonic output to the probe 2b. The driving signal is subjected to the constant current control by the current controller 14. Therefore, the power amplifier 15 preferably amplifies the voltage of the input driving signal and consequently amplifies the power thereof. The power amplifier 15 may amplify the power of the driving signal by receiving a signal corresponding to the current amplification factor from the current controller 14, and by amplifying the current and voltage of the driving signal based on the current amplification factor. In the latter case, the current controller 14 does not need to amplify the current of the driving signal.

The amplified driving signal is input to the detector 16. The detector 16 detects a current and a voltage supplied from the input driving signal to the ultrasonic transducer 2a. The detector 16 also generates the current phase signal $\theta_I$ corresponding to the phase of the detected current and the voltage phase signal $\theta_V$ corresponding to the phase of the detected voltage. The detector 16 further generates the current signal S3 corresponding to the current $|I|$ supplied to the ultrasonic transducer 2a and the voltage signal S4 corresponding to a voltage $|V|$ supplied to the ultrasonic transducer 2a. The detector 16 outputs the generated current phase signal $\theta_I$ to the switch circuit 12 and the controller 17, and outputs the generated voltage phase signal $\theta_V$ to the driver circuit 13 and the controller 17. That is, the controller 16 outputs the current phase signal $\theta_I$ and the voltage phase signal $\theta_V$ as a feedback signal fed back to the driver circuit 13, and outputs the current signal S3 as a feedback signal fed back to the current controller 14. Further, the detector 16 outputs the power-amplified driving signal to the ultrasonic transducer 2a. In this case, the ultrasonic transducer 2a converts an electric energy obtained by the input driving signal into the ultrasonic vibration, and outputs the ultrasonic vibration to the probe 2b.

The controller 17 is composed of, for example, a read only memory (ROM) which stores a processing program and various pieces of data, a random access memory (RAM) which stores various operation parameters and the like, and a central processing unit (CPU) which executes the processing program stored in the ROM. The controller 17 includes a resonance point detection unit 17a, an output control unit 17b, an impedance processing unit 17c, and a storage unit 17d. The storage unit 17d stores upper impedance limits R1 and R3 and a lower impedance limit R2 to be explained later. The controller 17 manages the upper impedance limits R1 and R3 and the lower impedance limit R2 as determination reference information. When the operator inputs the set ultrasonic output, the controller 17 stores and manages the input set ultrasonic output in the storage unit 17b as driving management information on driving control over the ultrasonic transducer 2a. In addition, the controller 17 stores and manages the current phase corresponding to the current phase signal $\theta_I$ and the voltage phase corresponding to the voltage phase signal $\theta_V$ as phase information on the phases of the current and the voltage of the driving signal. Further, the controller 17 stores and manages the current $|I|$ corresponding to the current signal S3 and the voltage $|V|$ corresponding to the voltage signal S4 as driving information on driving of the ultrasonic transducer 2a.

The controller 17 controls the switch circuit 12 to supply the reference frequency signal S1 to the driver circuit 13 when the ultrasonic transducer 2a is activated, and to supply the current phase signal $\theta_I$ to the driver circuit 13 when the resonance point detection unit 17a detects the resonance frequency fr. The controller 17 outputs an instruction signal to the output control unit 17b to instruct the output control unit 17b to reduce the set current $|I|_{set}$, and an instruction signal to the warning circuit 18 to instruct the warning circuit 18 to output a warning when determining that the probe 2b is in contact with the operation instrument. The warning circuit 18 is composed of, for example, a display circuit and a sound source circuit. The warning circuit 18 makes a display or outputs a buzzer which indicates a state in which the probe 2b is in contact with the operation instrument to the output section 1c of the controller device 1 according to the instruction signal input thereto from the controller 17. The controller 17 may output the instruction signal to warning circuit 18 to instruct the warning circuit 18 to output a warning when the ultrasonic vibration is excessively output to the treatment target such as the living tissue.

The resonance point detection unit 17a detects the resonance frequency fr of the ultrasonic transducer 2a based on phase information on both the current and the voltage of the driving signal input to the ultrasonic transducer 2a. It is noted that the resonance point detection unit 17a receives the current phase signal $\theta_I$ and the voltage phase signal $\theta_V$ from the detector 16, and acquires the phase information on both the current and voltage of the driving signal input to the ultrasonic transducer 2a. When recognizing that the difference between the current phase and the voltage phase of the driving signal is zero, the resonance point detection unit 17a detects the resonance frequency fr of the ultrasonic transducer 2a. In this case, the ultrasonic transducer 2a turns into a state in which the ultrasonic transducer 2a can be driven with the resonance frequency fr or the frequency near the resonance frequency fr. If the resonance point detection unit 17a detects the resonance frequency fr, the controller 17 controls the switch circuit 12 to input the current phase signal $\theta_I$ fed back from the detector 16 to the driver circuit 13 as already explained.

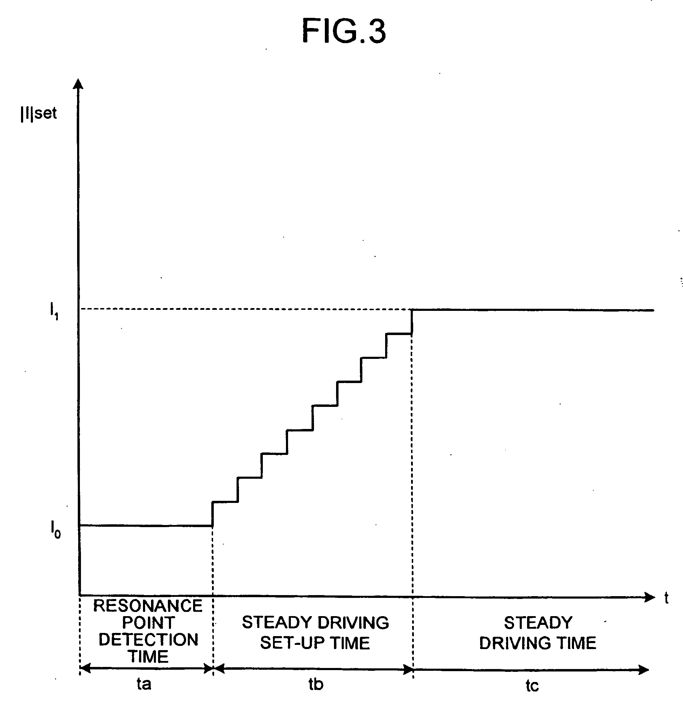

The output control unit 17b determines the set current $|I|_{set}$ of the driving signal based on the set ultrasonic output, and outputs the current setting signal S2 corresponding to the set current $|I|_{set}$ to the current controller 14. The output control unit 17b outputs the appropriate set current $|I|_{set}$ according to a state of the ultrasonic transducer 2a after being activated, and outputs the set current $|I|_{set}$ corresponding to the set ultrasonic output when the ultrasonic transducer 2a turns into the state in which the ultrasonic transducer 2a can be driven with the resonance frequency fr. Fig. 3 depicts a fluctuation in set current $|I|_{set}$ when the set current $|I|_{set}$ is set according to the state of the ultrasonic transducer 2a. As shown in Fig. 3, the output control unit 17b sets the set current $|I|_{set}$ at a set value $I_0$ at a time $t_a$, gradually increases the set current $|I|_{set}$ from the set value $I_0$ to a set value $I_1$ at a time $t_b$, and sets the set current $|I|_{set}$ at a set value $I_1$ at a time $t_c$.

The time $t_a$ is a time required until the resonance point detection unit 17a detects the resonance frequency fr of the ultrasonic transducer 2a (resonance point detection time). The time $t_b$ is a time required until the current of the driving signal input to the ultrasonic transducer 2a is amplified up to a current corresponding to the set ultrasonic output, i.e., a set-up time required until the ultrasonic transducer 2a turns into a state (steady driving state) in which the ultrasonic transducer 2a can stably drive the ultrasonic output corresponding to the set ultrasonic output. The time $t_c$ is a time at which the ultrasonic transducer 2a is in the steady driving state and can output a desired ultrasonic vibration. Namely, when the ultrasonic transducer 2a is in a state in which the ultrasonic transducer 2a cannot be driven with the resonance frequency, then the output control unit 17b sets the set current $|I|_{set}$ at the set value $I_0$ and drives the ultrasonic transducer 2a at a low current. When the ultrasonic transducer 2a turns into a state in which the ultrasonic transducer 2a can be driven with the resonance frequency fr, the output control unit 17b increases the set current $|I|_{set}$ which has been set at the set value $I_0$ to the set value $I_1$. Thereafter, when the output control unit 17b sets the set current $|I|_{set}$ at the set value $I_1$, the current $|I|$ corresponding to the set value $I_1$ is applied to the ultrasonic transducer 2a, thereby turning the ultrasonic transducer 2a into the steady driving state.

Further, when the set current $|I|_{set}$ corresponding to the set ultrasonic output is output, the output control unit 17b sets a threshold current $I_{th}$ for the current $|I|$ of the driving signal and monitors the current $|I|$. At this time, the controller 17 stores and manages the set threshold current $I_{th}$ as a part of the determination reference information in the storage unit 17d. It is noted that the threshold current $I_{th}$ is a value for determining whether the current of the driving signal is substantially equal to the set current $|I|_{set}$ corresponding to the set ultrasonic output. If the current $|I|$ is lower than the threshold current $I_{th}$, the current $|I|$ is determined to be lower than the set current $|I|set$ corresponding to the set ultrasonic output. Alternatively, the current controller 14 may monitor the current $|I|$ based on the threshold current $I_{th}$.

Further, when a mechanical load which may cause damage to the probe 2b is applied to the probe 2b, the output control unit 17b reduces the set current $|I|_{set}$ by as much as a predetermined current change amount $\Delta I_a$ under control of the controller 17. The output control unit 17b instructs the current control unit 14 to set the current $|I|$ of the driving signal to be lower than the set current $|I|_{set}$ corresponding to the set ultrasonic output. In this case, the output control unit 17b compares the threshold current $I_{th}$ with the current $|I|$, and determines whether the current $|I|$ is lower than the set current $|I|_{set}$ corresponding to the set ultrasonic output. When the mechanical load which may cause damage to the probe 2b is eliminated, the output control unit 17b increases the set current $|I|_{set}$ by as much as a predetermined current change amount $\Delta I_b$ under control of the controller 17. In this case, the output control unit 17b increases the set current $|I|_{set}$ up to the set current $|I|_{set}$ (e.g., set value $I_1$) corresponding to the set ultrasonic output.

The impedance processing unit 17c detects an impedance $|Z|$ of the ultrasonic transducer 2a when the ultrasonic transducer 2a is driven from the current and voltage of the driving signal input to the ultrasonic transducer 2a, and compares the obtained impedance $|Z|$ with the upper impedance limits R1 and R3 or with the lower impedance limit R2. Here, the impedance $|Z|$ corresponds to a second parameter, the upper impedance limits R1 and R3 correspond to a first parameter and a fourth parameter respectively, and the lower impedance limit R2 corresponds to a third parameter. It is noted that the impedance processing unit 17c receives the current signal S3 and the voltage signal S4 from the detector 16, and obtains the current and the voltage of the driving signal input to the ultrasonic transducer 2a. The controller 17 stores the detected impedance $|Z|$ as a part of the driving information in the storage unit 17b, and manages the impedance $|Z|$ as a comparison parameter to be compared with the upper impedance limits R1 and R3 and the lower impedance limit R2.

The upper impedance limit R1 and R3 are set as determination reference parameters for the impedance $|Z|$ in advance, and the lower impedance limit R2 is set as an output determination parameter for the impedance $|Z|$ in advance. Therefore, the upper impedance limit R1 is set within a range from an impedance equal to or higher than a highest impedance corresponding to the mechanical load exerted on the probe 2b caused by the contact of the probe 2b with the treatment target to an impedance equal to or lower than a lowest impedance corresponding to the mechanical load causing damage to the probe 2b.

The upper impedance limit R3 is the determination reference parameter for determining whether the mechanical load causing damage to the probe 2b is exerted on the probe 2b in an impedance comparing process carried out if amplitude modulation, to be explained later, is performed. Therefore, the upper impedance limit R3 is set within the impedance corresponding to the mechanical load exerted on the probe 2b caused by the contact of the probe 2b with the treatment target. Preferably, the upper impedance limit R3 is set within a range from an impedance lower than the highest impedance corresponding to the mechanical load exerted on the probe 2b caused by the contact of the probe 2b with the treatment target to an impedance equal to or higher than the highest impedance when the current of the driving signal to be amplitude-modulated is low.

The lower impedance limit R2 is the output determination parameter for determining whether the ultrasonic transducer which enables performing appropriate medical treatment to the treatment target is sufficiently output. Therefore, the lower impedance limit R2 is set within a range from an impedance equal to or higher than an impedance R0 with the resonance frequency fr of the probe 2b which is out of contact with the treatment target to an impedance equal to or lower than the lowest impedance corresponding to the mechanical load exerted on the probe 2b caused by the contact of the probe 2b with the treatment target.

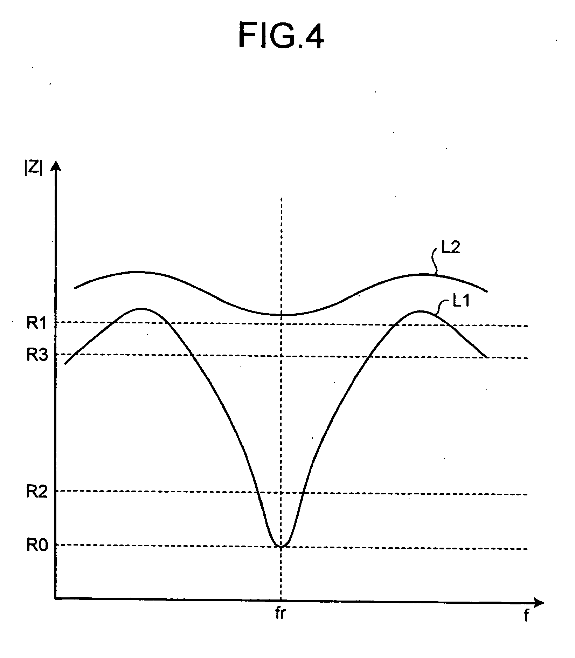

Fig. 4 is an example of the relationship between the impedance $|Z|$ and a frequency f when the ultrasonic transducer 2a is driven. Fig. 4 is an example of the upper impedance limits R1 and R3 and the lower impedance limit R2. In Fig. 4, a curve L1 shows an example of a fluctuation in the impedance $|Z|$ of the ultrasonic transducer 2a while the probe 2b is out of contact with the treatment target, and a curve L2 shows an example of a fluctuation in the impedance $|Z|$ of the ultrasonic transducer 2a while the probe 2b is in contact with the operation instrument. Each of the curves L1 and L2 takes a minimal when the frequency f is equal to the resonance frequency fr, and the minimal of the curve L1 is the impedance R0. The curve L2 always takes higher value than the curve L1 in a frequency range of the resonance frequency fr and frequencies near the resonance frequency fr. Namely, the impedance $|Z|$ of the ultrasonic transducer 2a when the ultrasonic transducer 2a is driven is a minimum with the resonance frequency fr irrespective of a contact state of the probe 2b, and rises when the mechanical load exerted on the probe 2b is increased by the contact of the probe 2b with the treatment target or the operation instrument. If the ultrasonic vibration is subjected to amplitude modulation, in particular, the mechanical load exerted on the probe 2b which transmits a low amplitude ultrasonic vibration is greatly increased when the probe 2b contacts with the operation instrument from the mechanical load when the probe 2b contacts with the treatment target.

By performing the comparing process for the impedance $|Z|$ based on this principle, the impedance processing unit 17c can determine a degree of the mechanical load exerted on the probe 2b. In addition, the controller 17 can determine whether the probe 2b is in contact with the operation instrument based on a result of the comparing process performed by the impedance processing unit 17c. For example, the curve L2 is present in a range in which the impedance $|Z|$ exceeds the upper impedance limit R1, so that it can be determined that the curve L2 corresponds to the impedance $|Z|$ of the ultrasonic transducer 2a when the probe 2b is in contact with the operation instrument.

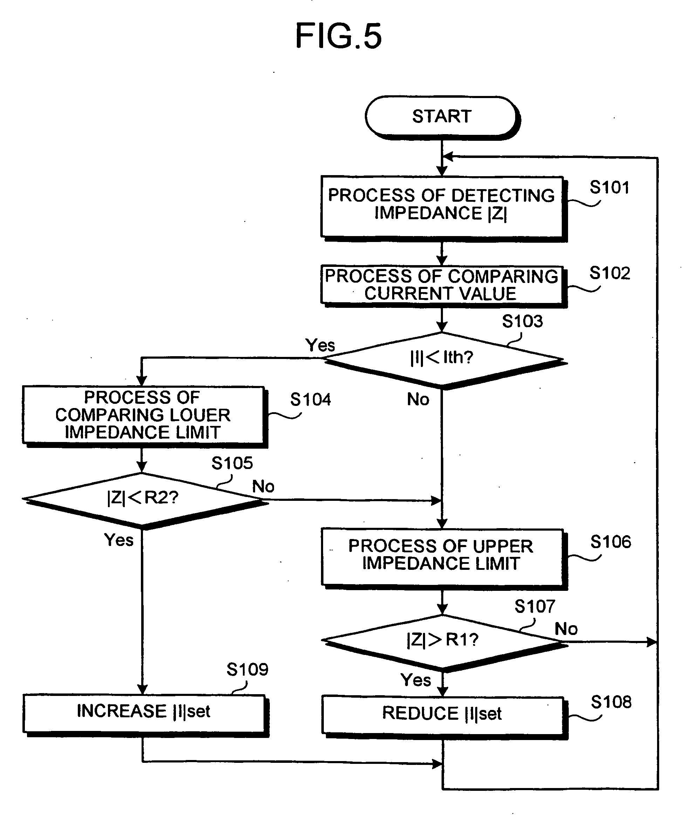

Fig. 5 is a flowchart of respective process procedures performed until the controller 17 determines whether the probe 2b is in contact with the operation instrument, and the controller 17 reduces the mechanical load exerted on the probe 2b based on this determination result or increases the reduced current of the driving signal. With reference to Fig.

5, the controller 17 receives the current signal S3 and the voltage signal S4 fed back from the detector 16, and the impedance processing unit 17c detects the current $|I|$ corresponding to the current signal S3 and the voltage $|V|$ corresponding to the voltage signal S4. The current $|I|$ and the voltage $|V|$ detected by the impedance processing unit 17c correspond to the current and the voltage of the driving signal for driving the ultrasonic transducer 2a, respectively. The impedance processing unit 17c operates and outputs the impedance $|Z|$ based on the detected current $|I|$ and voltage $|V|$, and thereby detects the impedance $|Z|$ of the ultrasonic transducer 2a when the ultrasonic transducer 2a is driven (at step S101). The impedance $|Z|$ can be operated by the following Equation (1).

$$|Z| = |V|/|I| \qquad\qquad\qquad ...(1)$$

When the impedance processing unit 17c detects the impedance $|Z|$, the output control unit 17b performs a current comparing process for comparing the current $|I|$ corresponding to the received current signal S3 with the threshold current $I_{th}$ (at step S102). If a result of this current comparing process indicates that the current $|I|$ is equal to or higher than the threshold current $I_{th}$ ("No" at step S103), then the output control unit 17b recognizes that the current $|I|$ corresponds to the set current $|I|_{set}$ corresponding to the set ultrasonic output. In addition, the controller 17 controls the impedance processing unit 17c to perform an upper impedance limit comparing process for comparing the impedance $|Z|$ with the upper impedance limit R1. Namely, the impedance processing unit 17c compares the detected impedance $|Z|$ with the upper impedance limit R1 (at step S106).

If a result of this upper impedance limit comparing process indicates that the impedance $|Z|$ is higher than the upper impedance limit R1 ("Yes" at step S107), the impedance processing unit 17c can determine that the mechanical load exerted on the probe 2b is a load which may cause damage to the probe 2b. The controller 17 can thereby determine that the probe 2b is in contact with the operation instrument. In this case, the controller 17 controls the output control unit 17b to reduce the set current $|I|_{set}$ by as much as the current change amount $\Delta I_a$. The output control unit 17b reduces the set current $|I|_{set}$ by as much as the current change amount $\Delta Ia$ under control of the controller 17 (at step S108), and outputs the current setting signal S2 corresponding to the reduced set current $|I|_{set}$ to the current controller 14. At this step, the current $|I|$ of the driving signal is controlled to be lower than the set current $|I|_{set}$ corresponding to the set ultrasonic output. It is thereby possible to reduce the amplitude of the ultrasonic vibration transmitted to the probe 2b, and reduce the mechanical load exerted on the probe 2b. If the current change amount $\Delta I_a$ is set large, the controller 17 can increase a change amount of the set current $|I|_{set}$. As a result, the controller 17 can promptly stop driving the ultrasonic transducer 2a when the probe 2b contacts with the operation instrument.

The controller repeats the respective process steps of step S101 and the following steps. In addition, if the result of the upper impedance limit comparing process performed by the impedance processing unit 17c indicates that the impedance $|Z|$ is equal to or lower than the upper impedance R1 ("No" at step S107), the impedance processing unit 17c can determine that the mechanical load exerted on the probe 2b is not a load which may cause damage to the probe 2b. In this case, the controller 17 repeats the respective process steps of step S101 and the following steps.

If the result of the current comparing process performed by the output control unit 17b indicates that the current $|I|$ is lower than the threshold current $I_{th}$ ("Yes" at step S103), the output control unit 17b recognizes that the current $|I|$ is constant-current controlled to be a set value lower than the set current $|I|_{set}$ corresponding to the set ultrasonic output. In addition, the controller 17 controls the impedance processing unit 17c to perform a lower impedance limit comparing process for comparing the impedance $|Z|$ with the lower impedance limit R2. Namely, the impedance processing unit 17c compares the detected impedance $|Z|$ with the lower impedance limit R2 (at step S104).

If a result of the lower impedance limit comparing process indicates that the impedance $|Z|$ is equal to or higher than the lower impedance limit R2 ("No" at step S105), the controller 17 performs the respective process steps of step S106 and the following steps. If the result of the lower impedance limit comparing process indicates that the impedance $|Z|$ is lower than the lower impedance limit R2 ("Yes" at step S105), the impedance processing unit 17c determines that the ultrasonic vibration which enables performing an appropriate medical treatment to the treatment target is not sufficiently output from the ultrasonic transducer 2a. In addition, the controller 17 controls the output control unit 17b to increase the set current $|I|_{set}$ by as much as the current change amount $\Delta I_b$. The output control unit 17b increases the set current $|I|_{set}$ by as much as the current change amount $\Delta I_b$ under control of the controller 17 (at step S109), and outputs the current setting signal S2 corresponding to the increased set current $|I|_{set}$ to the current controller 14. In this case, the current $|I|$ of the driving signal is controlled to be increased up to the set current $|I|_{set}$ corresponding to the ultrasonic output set by the operator. The ultrasonic transducer 2a can restore the amplitude of the ultrasonic vibration which has been reduced once to the original amplitude, and output the ultrasonic vibration which enables performing the appropriate medical treatment to the treatment target. It is noted that if the current change amount $\Delta I_b$ is set large, the controller 17 can increase the change amount of the set current $|I|_{set}$. As a result, if the amplitude of the ultrasonic vibration which has been once reduced is insufficient to carry out the medical treatment, the controller 17 can control the ultrasonic transducer 2a to restore its ultrasonic vibration output at early timing. Thereafter, the controller 17 repeats the respective process steps of step S101 and the following steps.

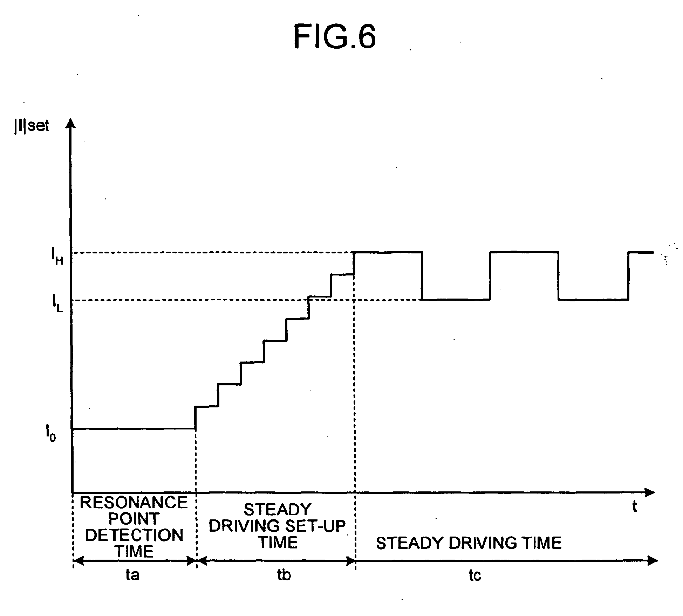

In the ultrasonic output of the ultrasonic vibrato 2a, the current $|I|$ of the driving signal input to the ultrasonic transducer 2a in the steady driving state is often changed to thereby modulate the amplitude of the ultrasonic vibration output from the ultrasonic transducer 2a. Fig. 6 is an example of a fluctuation in the set current $|I|_{set}$ set by the output control unit 17b. As shown in Fig. 6, the output control unit 17b sets the set current $|I|_{set}$ at the set value $I_0$ at the time $t_a$, and gradually increases the set current $|I|_{set}$ from the set value $I_o$ to a set value $I_H$ at the time $t_b$. The output control unit 17b alternately outputs the set value $I_H$ and a set value $I_L$ as the set current $|I|_{set}$ at the time $t_c$. Namely, similarly to the instance in which the amplitude modulation is not performed as shown in Fig. 3, the output control unit 17b increases the set current $|I|_{set}$ to the set value $I_H$, and then alternatively sets the set value $I_H$ and the set value $I_L$ as the set current $|I|_{set}$.

In this case, the ultrasonic transducer 2a alternately receives the driving signal at a current $|I|_H$ corresponding to the set value $I_H$ and the driving signal at a current $|I|_L$ corresponding to the set value $I_L$. The ultrasonic transducer 2a can thereby output the amplitude-modulated ultrasonic vibration to the probe 2b. The set value $I_H$ is a value higher than the set value $I_L$. A difference between the set values $I_H$ and $I_L$ corresponds to a percentage modulation of the amplitude modulation. If this difference is set constant, the ultrasonic transducer 2a outputs the ultrasonic vibration which has been subjected to certain amplitude modulation.

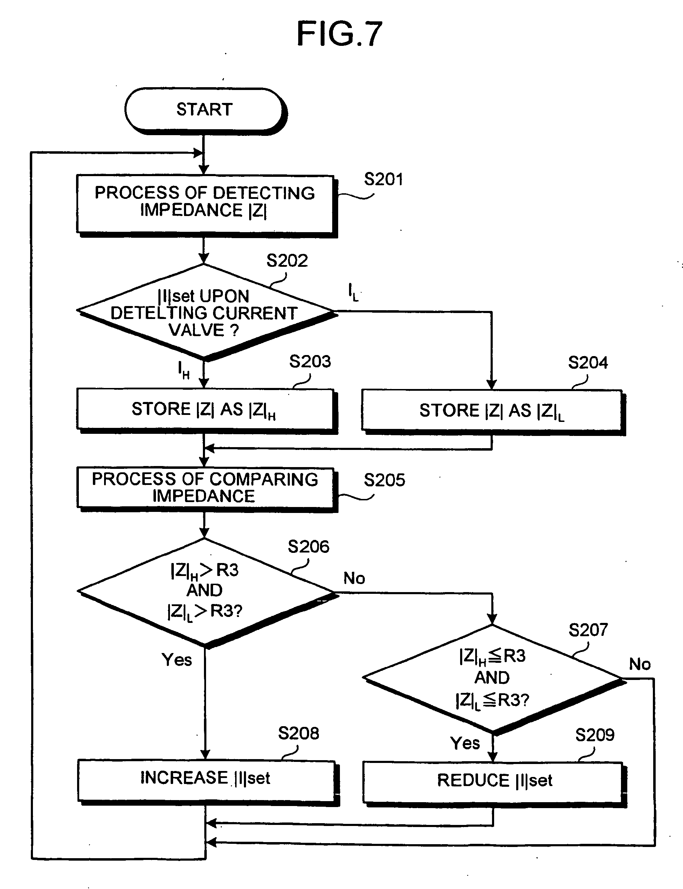

Fig. 7 is a flowchart of process procedures performed until the controller 17 determines whether the probe 2b is in contact with the operation instrument, reduces the mechanical load exerted on the probe 2b or increases the reduced current of the driving signal based on a result of this determination if the ultrasonic transducer 2a outputs the amplitude-modulated ultrasonic vibration. With reference to Fig. 7, the controller 17 receives the current signal S3 and the voltage signal S4 fed back from the detector 16, and the impedance processing unit 17c detects a current corresponding to the current signal S3 and a voltage corresponding to the voltage signal S4. The impedance processing unit 17c then operates and outputs an impedance based on the current corresponding to the current signal S3 and the voltage corresponding to the voltage signal S4, and thereby detects the impedance $|Z|$ of the ultrasonic transducer 2a when the ultrasonic transducer 2a is driven (at step S201).

If the output control unit 17b outputs a set value $I_H$ as the set current $|I|_{set}$ of the driving signal, the current of the driving signal input to the ultrasonic transducer 2a is the current $|I|_H$ corresponding to the set value $I_H$. The controller 17 recognizes that the set current $|I|_{set}$ of the driving signal is the set value $I_H$. In addition, the impedance processing unit 17c detects the current $|I|_H$ of the driving signal corresponding to the set value $I_H$ by receiving the current signal S3 as explained above ("$I_H$" at step S202), and detects the voltage $|V|_H$ of the driving signal by receiving the voltage signal S4 as explained above. Here, the voltage $|V|_H$ corresponds to a fifth parameter In this case, the impedance $|Z|$ detected at step S201 is an impedance $|Z|_H$ operated and output based on the current $|I|_H$ and the voltage $|V|_H$. The controller 17 stores and manages the impedance $|Z|_H$ as part of the driving information in the storage unit 17d (at step S204).

If the output control unit 17b outputs the set value $I_L$ as a set current $|I|_{set}$ of the driving signal, the current of the driving signal input to the ultrasonic transducer 2a is the current $|I|_L$ corresponding to the set value $I_L$. The controller 17 recognizes that the set current $|I|$set of the driving signal is the set value $I_L$. In addition, the impedance processing unit 17c detects the current $|I|_L$ of the driving signal corresponding to the set value $I_L$ by receiving the current signal S3 as explained above ("$I_L$" at step S202), and detects the voltage $|V|_L$ of the driving signal by receiving the voltage signal S4 as explained above. Here, the voltage $|V|_L$ corresponds to a sixth parameter. In this case, the impedance $|Z|$ detected at step S201 is an impedance $|Z|_L$ operated and output based on the current $|I|_L$ and the voltage $|V|_L$. The controller 17 stores and manages the impedance $|Z|_L$ as part of the driving information in the storage unit 17d (at step S203).

The voltage $|V|_H$ is a voltage when the driving signal at the current $|I|_H$ is amplified to a desired power, and the voltage $|V|_L$ is a voltage when the driving signal at the current $|I|_L$ is amplified to a desired power. At step S201, the impedance processing unit 17c operates and outputs the impedances $|Z|_H$ and $|Z|_L$ by the following Equations (2) and (3), respectively.

$$|Z|_H = |V|_H / |I|_H \qquad \qquad ...(2)$$

$$|Z|_L = |V|_L / |I|_L \qquad \qquad ...(3)$$

The impedance processing unit 17c then performs an impedance comparing process for comparing the detected impedance $|Z|_H$ or $|Z|_L$ with the upper impedance limit R3 (at step S205). If a result of this impedance comparing process indicates that at least one of the impedances $|Z|_H$ and $|Z|_L$ does not satisfy a condition that the impedance is higher than the upper impedance limit R3 ("No" at step S206), and that at least one of the impedances $|Z|_H$ and $|Z|_L$ does not satisfy a condition that the impedance is equal to or lower than the upper impedance limit R3 ("No" at step S207), the controller 17 repeats the respective process steps of step S201 and the following steps.

If the result of the impedance comparing process at step S205 indicates that each of the impedances $|Z|_H$ and $|Z|_L$ satisfies the condition that the impedance is higher than the upper impedance limit R3 ("Yes" at step S206), the impedance processing unit 17c can determine that the mechanical load exerted on the probe 2b is a load which may cause damage to the probe 2b. In addition, the controller 17 can thereby determine that the probe 2b is in contact with the operation instrument. In this case, the controller 17 controls the output control unit 17b to reduce the set current $|I|$set by as much as the current change amount $\Delta I_a$. Accordingly, the output control unit 17b reduces the set value $I_H$ or $I_L$ by as much as the current change amount $\Delta I_a$ similarly to step S108 (at step S208), and outputs the set current signal S2 corresponding to the reduced set value $I_H$ or $I_L$ to the current controller 14. As a result, the amplitude of the ultrasonic vibration transmitted to the probe 2b can be reduced, and the mechanical load exerted on the probe 2b can be reduced. Thereafter, the controller 17 repeats the respective process steps of step S201 and the following steps.

If the result of the impedance comparing process at step S205 indicates that at least one of the impedances $|Z|_H$ and $|Z|_L$ does not satisfy the condition that the impedance is higher than the upper impedance limit R3 ("No" at step S206), and that each of the impedances $|Z|_H$ and $|Z|_L$ satisfies the condition that the impedance is equal to or lower than the upper impedance limit R3 ("Yes" at step S207), the impedance processing unit 17c determines that the ultrasonic vibration which enables performing an appropriate medical treatment to the treatment target is not sufficiently output from the ultrasonic transducer 2a. In addition, the controller 17 controls the output control unit 17b to increase the set current $|I|_{set}$ by as much as the current change amount $\Delta I_b$. Accordingly, the output control unit 17b increases the set values $I_H$ and $I_L$ each by as much as the current change amount $\Delta I_b$ similarly to step S109 (at step S209), and outputs the current set signal S2 corresponding to the increased set value $I_H$ or $I_L$ to the current controller 14. As a result, the ultrasonic transducer 2a can restore the amplitude of the ultrasonic vibration which has been reduced once to the original amplitude, and output the ultrasonic vibration which enables performing the appropriate medical treatment to the treatment target. Thereafter, the controller 17 repeats the respective process steps of step S201 and the following steps.

According to the first embodiment, if the amplitude-modulated ultrasonic vibration is output, both the currents $|I|_H$ and $|I|_L$ of the driving signal to be amplitude-modulated are reduced, thereby reducing the mechanical load exerted on the probe 2b. However, a method for reducing the mechanical load according to the present invention is not limited to this method. The mechanical load exerted on the probe 2b may be reduced by reducing the difference between the currents $|I|_H$ and $|I|_L$, i.e., reducing the percentage modulation of the amplitude modulation.

According to the first embodiment, the impedance of the ultrasonic vibration driven with the resonance frequency is detected based on the current and the voltage detected from the driving signal input to the ultrasonic transducer 2a. The impedance is compared with the preset upper impedance limit. The driving of the ultrasonic transducer 2a is controlled according to the result of the comparing process, and the amplitude of the ultrasonic vibration output to the probe 2b is changed. Specifically, when the impedance $|Z|$ of the ultrasonic transducer 2a is higher than the upper impedance limit R1 shown in Fig. 4, it is determined that the mechanical load which may cause damage to the probe 2b is exerted on the probe 2b due to the contact of the probe 2b with the operation instrument. In addition, the controller 17 exercises driving control for reducing the current supplied to the ultrasonic transducer 2a, and reduces the amplitude of the ultrasonic vibration output to the probe 2b. Therefore, the contact of the probe 2b with the operation instrument can be instantly detected, and the mechanical load exerted on the probe 2b can be reduced before the probe 2b is severely damaged. It is thereby possible to prevent damage to the probe 2b which may occur while the medical treatment on the treatment target is performed.

Further, according to the first embodiment, the detected impedance of the ultrasonic transducer 2a is compared with the lower impedance limit. The driving of the ultrasonic transducer 2a is controlled according to the result of the comparing process, and the amplitude of the ultrasonic vibration output to the probe 2a is changed. Specifically, if the impedance $|Z|$ of the ultrasonic transducer 2a is within the range from the lower impedance limit R2 to the upper impedance limit R1 shown in Fig. 4, then it is determined that the mechanical load is eliminated, and that the ultrasonic vibration which enables performing the appropriate medical treatment is output from the ultrasonic transducer 2a. In addition, the controller 17 controls driving of the ultrasonic transducer 2a to keep a present state. If this impedance $|Z|$ is lower than the lower impedance limit R2, then it is determined that the ultrasonic vibration which enables performing the appropriate medical treatment is not output from the ultrasonic transducer 2a despite the elimination of the mechanical load. In addition, the controller 17 exercises driving control for increasing the current supplied to the ultrasonic transducer 2a, and restores the amplitude of the ultrasonic vibration output to the probe 2b to the original amplitude. Therefore, it is possible to ensure that the ultrasonic vibration for performing the appropriate medical treatment on the treatment target is output. The damage to the probe 2b which may occur while the medical treatment is performed can be prevented, and an operating efficiency of the medical treatment can be improved.

Meanwhile, if the amplitude-modulated ultrasonic vibration is to be output from the probe 2b, the respective impedances of the ultrasonic vibration driven with the resonance frequency are detected for the high current and the low current of the driving signal for attaining the amplitude modulation similarly to the instance in which the amplitude of the ultrasonic vibration is not modulated. In addition, the respective impedances thus obtained are compared with the upper impedance limit R3 set in advance. The driving of the ultrasonic transducer is controlled according to the result of the comparing process, and the amplitude of the ultrasonic vibration output to the probe is changed. Therefore, the same functions and advantages as those of the instance in which the amplitude of the ultrasonic vibration is not modulated can be attained.

A second embodiment of the present invention will be explained below. According to the first embodiment, the impedance of the ultrasonic transducer when the transducer is driven is detected and the comparing process is carried out for the impedance. Whereas according to the second embodiment, a driving power for driving the ultrasonic transducer is detected, and a comparing process is carried out for the driving power.

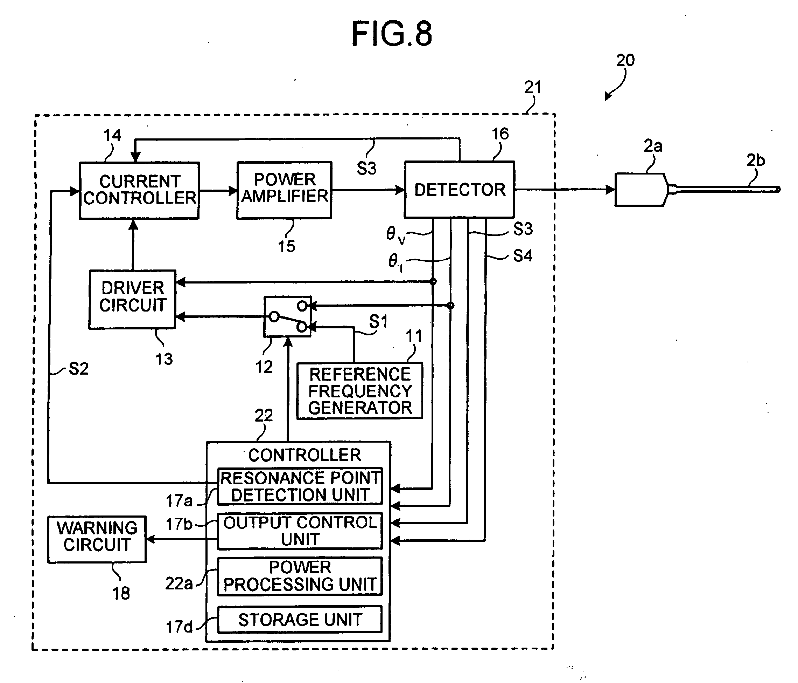

Fig. 8 is a block diagram which depicts an example of the basic configuration of a control device of an ultrasonic surgical system according to the second embodiment of the present invention. In a control device 21 of an ultrasonic surgical system 20, a power processing unit 22a is provided in place of the impedance processing unit 17c in the controller 17 arranged in the control device 1 of the ultrasonic surgical system 10 according to the first embodiment. The other constituent parts of the ultrasonic surgical system 20 are identical to those of the ultrasonic surgical system 10, and like parts are designated with like reference signs.

The power processing unit 22a of a controller 22 detects a driving power |W| of the ultrasonic transducer 2a based on a current and a voltage of a driving signal input to the ultrasonic transducer 2a. In addition, the power processing unit 22a performs a comparing process for comparing the detected driving power |W| with upper power limits W1 and W3 or with a lower power limit W2. Here, the driving power |W| corresponds to the second parameter, the upper power limits W1 and W3 correspond to the first parameter and the fourth parameter respectively, and the lower power limit W2 corresponds to the third parameter. The power processing unit 22a receives the current signal S3 and the voltage signal S4 from the detector 16, and obtains the current and voltage of the driving signal input to the ultrasonic transducer 2a. The controller 22 stores the detected driving power |W| as a part of driving information in the storage unit 17d, and manages the driving power |W| as a comparison parameter to be compared with the upper power limits W1 and W3 and the lower power limit W2.

The upper power limits W1 and W3 are set as determination reference parameters for the driving power |W| in advance, and the lower power limit W2 is set as an output determination parameter for the driving power |W| in advance. The controller 22 stores and manages the upper power limits W1 and W3 and the lower power limit W2 as determination reference information in the storage unit 17d. Therefore, the upper power limit W1 is set within a range from a power equal to or higher than a highest power corresponding to the mechanical load exerted on the probe 2b caused by the contact of the probe 2b with the treatment target to a power equal to or lower than a lowest power corresponding to the mechanical load causing damage to the probe 2b.

The upper power limit W3 is the determination reference parameter for determining whether the mechanical load causing damage to the probe 2b is exerted on the probe 2b in a power comparing process carried out if amplitude modulation, to be explained later, is performed. Therefore, the upper power limit W3 is set within the power corresponding to the mechanical load exerted on the probe 2b caused by the contact of the probe 2b with the treatment target. Preferably, the upper power limit W3 is set within a range from a power lower than the highest power corresponding to the mechanical load exerted on the probe 2b caused by the contact of the probe 2b with the treatment target to a power equal to or higher than the highest power when the current of the driving signal to be amplitude-modulated is low.

The lower power limit W2 is the output determination parameter for determining whether the ultrasonic transducer which enables performing appropriate medical treatment to the treatment target is sufficiently output. Therefore, the lower power limit W2 is set within a range from a power equal to or higher than a power W0 with the resonance frequency fr of the probe 2b which is out of contact with the treatment target to a power equal to or lower than the lowest power corresponding to the mechanical load exerted on the probe 2b caused by the contact of the probe 2b with the treatment target.

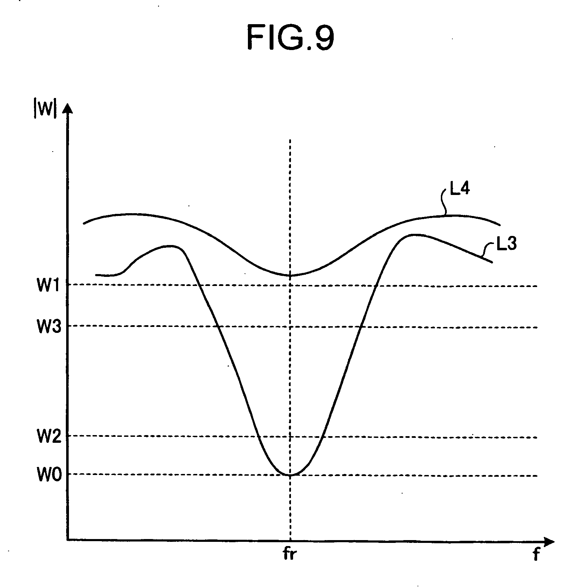

Fig. 9 depicts an example of the relationship between the power |W| and the frequency f when the ultrasonic transducer 2a is driven. Fig. 9 depicts examples of the upper power limits W1 and W3 and the lower power limit W2. In Fig. 9, a curve L3 shows an example of a fluctuation in the power |W| of the ultrasonic transducer 2a while the probe 2b is out of contact with the treatment target, and a curve L4 shows an example of a fluctuation in the power |W| of the ultrasonic transducer 2a while the probe 2b is in contact with the operation instrument. Each of the curves L3 and L4 takes a minimal when the frequency f is equal to the resonance frequency fr, and the minimal of the curve L3 is the power W0. The curve L4 always takes higher value than the curve L3 in a frequency range of the resonance frequency fr and frequencies near the resonance frequency fr. Namely, the power |W| of the ultrasonic transducer 2a when the ultrasonic transducer 2a is driven is a minimum with the resonance frequency fr irrespective of a contact state of the probe 2b, and rises when the mechanical load exerted on the probe 2b is increased by the contact of the probe 2b with the treatment target or the operation instrument. If the ultrasonic vibration is subjected to amplitude modulation, in particular, the mechanical load exerted on the probe 2b which transmits a low amplitude ultrasonic vibration is greatly increased when the probe 2b contacts with the operation instrument from the mechanical load when the probe 2b contacts with the treatment target.

By performing the comparing process for the power |W| based on this principle, the power processing unit 22c can determine a degree of the mechanical load exerted on the probe 2b. In addition, the controller 22 can determine whether the probe 2b is in contact with the operation instrument based on a result of the comparing process performed by the power processing unit 22a. For example, the curve L4 is present in a range in which the power |W| exceeds the upper power limit W1, so that it can be determined that the curve L4 corresponds to the power |W| of the ultrasonic transducer 2a when the probe 2b is in contact with the operation instrument.

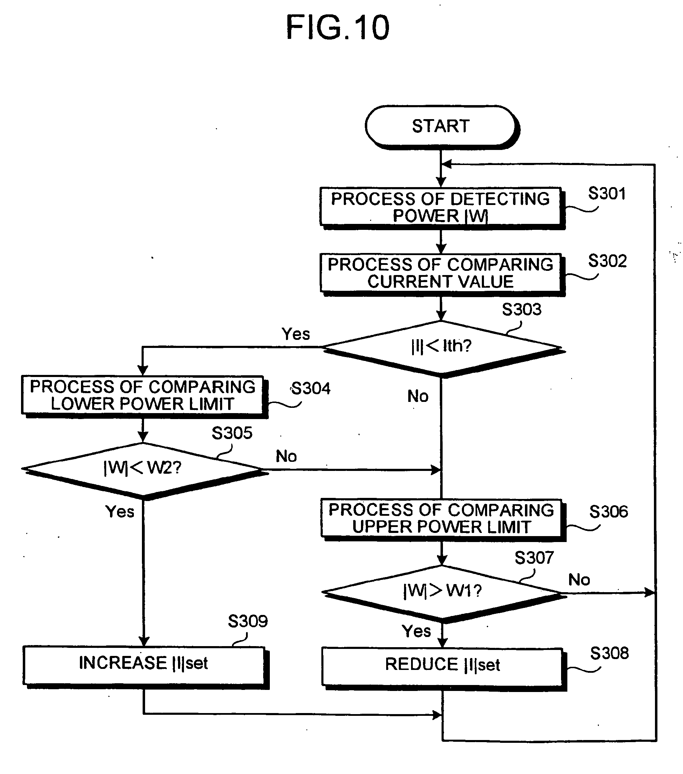

Fig. 10 is a flowchart of respective process procedures performed until the controller 22 determines whether the probe 2b is in contact with the operation instrument, and the controller 22 reduces the mechanical load exerted on the probe 2b based on this determination result or increases the reduced current of the driving signal. With reference to Fig. 10, the controller 22 receives the current signal S3 and the voltage signal S4 fed back from the detectoW16, and the power processing unit 22a detects the current |I| corresponding to the current signal S3 and the voltage |V| corresponding to the voltage signal S4. The current |I| and the voltage |V| detected by the power processing unit 22a correspond to the current and the voltage of the driving signal for driving the ultrasonic transducer 2a, respectively. The power processing unit 22a operates and outputs the power |W| based on the detected current |I| and voltage |V|, and thereby detects the power |W| of the ultrasonic transducer 2a when the ultrasonic transducer 2a is driven (at step S301). The power |W| can be operated by the following Equation (4).

$$|W| = |V|/|I| \qquad\qquad ...(4)$$

When the power processing unit 22a detects the power |W|, the output control unit 17b performs a current comparing process similarly to step S102 (at step S302). If a result of this current comparing process indicates that the current |I| is equal to or higher than the threshold current $I_{th}$ ("No" at step S303), then the output control unit 17b recognizes that the current |I| corresponds to the set current |I|set corresponding to the set ultrasonic output similarly to the first embodiment. In addition, the controller 22 controls the power processing unit 22a to perform an upper power limit comparing process for comparing the power |W| with the upper power limit W1. Namely, the power processing unit 22a compares the detected power |W| with the upper power limit W1 (at step S306).

If a result of this upper power limit comparing process indicates that the power |W| is higher than the upper power limit W1 ("Yes" at step S306), the power processing unit 22a can determine that the mechanical load exerted on the probe 2b is a load which may cause damage to the probe 2b. The controller 22 can thereby determine that the probe 2b is in contact with the operation instrument. In this case, the controller 22 controls the output control unit 17b to reduce the set current $|I|_{set}$ by as much as the current change amount $\Delta I_a$. The output control unit 17b reduces the set current $|I|_{set}$ by as much as the current change amount $\Delta I_a$ under control of the controller 22 (at step S308), and outputs the current setting signal S2 corresponding to the reduced set current $|I|_{set}$ to the current controller 14. At this step, the current |I| of the driving signal is controlled to be lower than the set current $|I|_{set}$ corresponding to the set ultrasonic output. It is thereby possible to reduce the amplitude of the ultrasonic vibration transmitted to the probe 2b, and reduce the mechanical load exerted on the probe 2b.

The controller repeats the respective process steps of step S301 and the following steps. In addition, if the result of the upper power limit comparing process performed by the power processing unit 22a indicates that the power |W| is equal to or lower than the upper power W1 ("No" at step S307), the power processing unit 22a can determine that the mechanical load exerted on the probe 2b is not a load which may cause damage to the probe 2b. In this case, the controller 22 repeats the respective process steps of step S301 and the following steps. If the result of the current comparing process performed by the output control unit 17b indicates that the current |I| is lower than the threshold current $I_{th}$ ("Yes" at step S303), the output control unit 17b recognizes that the current |I| is constant-current controlled to be a set value lower than the set current $|I|_{set}$ corresponding to the set ultrasonic output similarly to the first embodiment. In addition, the controller 22 controls the power processing unit 22a to perform a lower power limit comparing process for comparing the power |W| with the lower power limit W2. Namely, the power processing unit 22a compares the detected power |W| with the lower power limit W2 (at step S304).

If a result of the lower power limit comparing process indicates that the power |W| is equal to or higher than the lower power limit W2 ("No" at step S305), the controller 22 performs the respective process steps of step S306 and the following steps. If the result of the lower power limit comparing process indicates that the power |W| is lower than the lower power limit W2 ("Yes" at step S305), the power processing unit 22a determines that the ultrasonic vibration which enables performing an appropriate medical treatment to the treatment target is not sufficiently output from the ultrasonic transducer 2a. In addition, the controller 22 controls the output control unit 17b to increase the set current $|I|_{set}$ by as much as the current change amount $\Delta I_b$ similarly to the first embodiment. The output control unit 17b increases the set current $|I|_{set}$ by as much as the current change amount $\Delta I_b$ under control of the controller 22 (at step S309), and outputs the current setting signal S2 corresponding to the increased set current $|I|_{set}$ to the current controller 14. In this case, the current $|I|$ of the driving signal is controlled to be increased up to the set current $|I|_{set}$ corresponding to the ultrasonic output set by the operator. The ultrasonic transducer 2a can thereby restore the amplitude of the ultrasonic vibration which has been reduced once to the original amplitude, and output the ultrasonic vibration which enables performing the appropriate medical treatment to the treatment target. Thereafter, the controller 22 repeats the respective process steps of step S301 and the following steps.

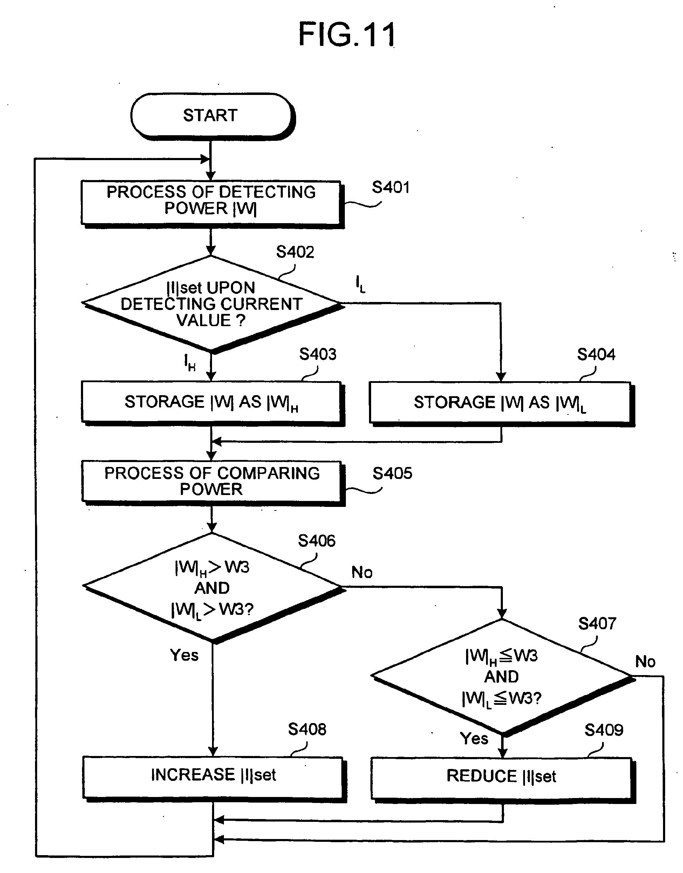

Fig. 11 is a flowchart of process procedures performed until the controller 22 determines whether the probe 2b is in contact with the operation instrument, reduces the mechanical load exerted on the probe 2b or increases the reduced current of the driving signal based on a result of this determination if the ultrasonic transducer 2a outputs the amplitude-modulated ultrasonic vibration. With reference to Fig. 11, the controller 22 receives the current signal S3 and the voltage signal S4 fed back from the detectoW16, and the power processing unit 22a detects a current corresponding to the current signal S3 and a voltage corresponding to the voltage signal S4. The power processing unit 22a then operates and outputs a power based on the current corresponding to the current signal S3 and the voltage corresponding to the voltage signal S4, and thereby detects the power $|W|$ of the ultrasonic transducer 2a when the ultrasonic transducer 2a is driven (at step S401).

If the controller 22 recognizes that the set current $|I|_{set}$ of the driving signal is the set value $I_H$ similarly to the first embodiment, the power processing unit 22a detects the current $|I|_H$ of the driving signal corresponding to the set value $I_H$ by receiving the current signal S3 as explained above ("$I_H$" at step S402), and detects the voltage $|V|_H$ of the driving signal by receiving the voltage signal S4 as explained above. In this case, the power $|W|$ detected at step S401 is a power $|W|_H$ operated and output based on the current $|I|_H$ and the voltage $|V|_H$. Here, the power $|W|_H$ corresponds to the fifth parameter. The controller 22 stores and manages the power $|W|_H$ as part of the driving information in the storage unit 17d (at step S403).

If the output control unit 17b recognizes that the set current $|I|_{set}$ of the driving signal is the set value $I_L$ similarly to the first embodiment, the power processing unit 22a detects the current $|I|_L$ of the driving signal corresponding to the set value $I_L$ by receiving the current signal S3 as explained above ("$I_L$" at step S402), and detects the voltage $|V|_L$ of the driving signal by receiving the voltage signal S4 as explained above. In this case, the power $|W|$ detected at step S401 is a power $|W|_L$ operated and output based on the current $|I|_L$ and the voltage $|V|_L$. Here, the power $|W|_L$ corresponds to the sixth parameter. The controller 22 stores and manages the power $|W|_L$ as part of the driving information in the storage unit 17d (at step S403).

At step S401, the power processing unit 22a operates and outputs the powers $|W|_H$ and $|W|_L$ by the following Equations (5) and (6), respectively.

$$|W|_H = |V|_H / |I|_H \qquad \qquad \dots (5)$$

$$|W|_L = |V|_L / |I|_L \qquad \qquad \dots (6)$$

The power processing unit 22a then performs a power comparing process for comparing the detected power $|W|_H$ or $|W|_L$ with the upper power limit W3 (at step S405). If a result of this power comparing process indicates that at least one of the powers $|W|_H$ and $|W|_L$ does not satisfy a condition that the power is higher than the upper power limit W3 ("No" at step S406), and that at least one of the powers $|W|_H$ and $|W|_L$ does not satisfy a condition that the power is equal to or lower than the upper power limit W3 ("No" at step S407), the controller 22 repeats the respective process steps of step S401 and the following steps.

If the result of the power comparing process at step 5405 indicates that each of the powers $|W|_H$ and $|W|_L$ satisfies the condition that the power is higher than the upper power limit W3 ("Yes" at step S406), the power processing unit 22a can determine that the mechanical load exerted on the probe 2b is a load which may cause damage to the probe 2b. In addition, the controller 22 can thereby determine that the probe 2b is in contact with the operation instrument. In this case, the controller 22 controls the output control unit 17b to reduce the set current $|I|_{set}$ by as much as the current change amount $\Delta Ia$. Accordingly, the output control unit 17b reduces the set value $I_H$ or $I_L$ by as much as the current change amount $\Delta I_a$ similarly to step S308 (at step S408), and outputs the set current signal S2 corresponding to the reduced set value $I_H$ or $I_L$ to the current controller 14. As a result, the amplitude of the ultrasonic vibration transmitted to the probe 2b can be reduced, and the mechanical load exerted on the probe 2b can be reduced. Thereafter, the controller 22 repeats the respective process steps of step S401 and the following steps.

If the result of the power comparing process at step S405 indicates that at least one of the powers $|W|_H$ and $|W|_L$ does not satisfy the condition that the power is higher than the upper power limit W3 ("No" at step S406), and that each of the powers $|W|_H$ and $|W|_L$ satisfies the condition that the power is equal to or lower than the upper power limit W3 ("Yes" at step S407), the power processing unit 22a determines that the ultrasonic vibration which enables performing an appropriate medical treatment to the treatment target is not sufficiently output from the ultrasonic transducer 2a. In addition, the controller 22 controls the output control unit 17b to increase the set current $|I|_{set}$ by as much as the current change amount $\Delta I_b$. Accordingly, the output control unit 17b increases the set values $I_H$ and $I_L$ each by as much as the current change amount $\Delta I_b$ similarly to step S209 (at step S409), and outputs the current set signal S2 corresponding to the increased set value $I_H$ or $I_L$ to the current controller 14. As a result, the ultrasonic transducer 2a can restore the amplitude of the ultrasonic vibration which has been reduced once to the original amplitude, and output the ultrasonic vibration which enables performing the appropriate medical treatment to the treatment target. Thereafter, the controller 22 repeats the respective process steps of step S401 and the following steps.

According to the second embodiment, the power of the ultrasonic vibration driven with the resonance frequency is detected based on the current and the voltage detected from the driving signal input to the ultrasonic transducer 2a. The power is compared with the preset upper power limit. If the result of the comparing process indicates that the mechanical load which may cause damage to the probe 2b is exerted on the probe 2b, the driving control for reducing the current supplied to the ultrasonic transducer 2a is exercised, and the amplitude of the ultrasonic vibration output to the probe 2b is reduced. Therefore, the contact of the probe 2b with the operation instrument can be instantly detected, and the mechanical load exerted on the probe 2b can be reduced before the probe 2b is severely damaged. Thus, the second embodiment exhibit the same functions and advantages as those of the first embodiment.

Further, according to the second embodiment, the detected power of the ultrasonic transducer 2a is compared with the lower power limit. If the result of the comparing process indicates that the mechanical load is eliminated, and that the ultrasonic vibration which enables performing the appropriate medical treatment is output from the ultrasonic transducer 2a, the ultrasonic transducer 2a is controlled to be driven to keep a present state. If the result of the comparing process indicates that the ultrasonic vibration which enables performing the appropriate medical treatment is not output from the ultrasonic transducer 2a despite the elimination of the mechanical load, driving control for increasing the current supplied to the ultrasonic transducer 2a is exercised, and the amplitude of the ultrasonic vibration output to the probe 2b is restored to the original amplitude. Therefore, it is possible to ensure that the ultrasonic vibration for performing the appropriate medical treatment on the treatment target is output. Thus, the second embodiment exhibits the same functions and advantages as those of the first embodiment.

Meanwhile, if the amplitude-modulated ultrasonic vibration is to be output from the probe 2b, the respective powers of the ultrasonic vibration driven with the resonance frequency are detected for the high current and the low current of the driving signal for attaining the amplitude modulation similarly to the instance in which the amplitude of the ultrasonic vibration is not modulated. In addition, the respective powers thus obtained are compared with the upper power limit W3 set in advance. The driving of the ultrasonic transducer is controlled according to the result of the comparing process, and the amplitude of the ultrasonic vibration output to the probe is changed. Therefore, the same functions and advantages as those of the instance in which the amplitude of the ultrasonic vibration is not modulated can be attained.

According to the first embodiment, the impedance of the ultrasonic transducer 2a when the ultrasonic transducer 2a is driven is detected based on the current and the voltage of the driving signal for driving the ultrasonic transducer 2a. The comparing process is performed for the detected impedance, and a fluctuation in the impedance relative to the preset determination reference is detected, thereby determining the mechanical load exerted on the probe 2a. According to the second embodiment, the driving power of the ultrasonic transducer 2a is detected based on the current and the voltage of the driving signal for driving the ultrasonic transducer 2a, the comparing process is performed for the detected driving power, and a fluctuation in the driving power relative to the preset determination reference, thereby determining the mechanical load exerted on the probe 2b.

If the driving of the ultrasonic transducer 2a to output the ultrasonic vibration is subjected to a constant-current control, the impedance and the driving power when this ultrasonic transducer 2a is driven are proportional to the driving voltage supplied to the ultrasonic transducer 2a. Namely, the driving voltage similarly changes similarly to the impedance or the driving power to correspond to an increase or a reduction of the mechanical load exerted on the probe 2b. Therefore, if a voltage comparing processing unit that detects the voltage of the driving signal from the driving signal input to the ultrasonic transducer 2a, and that performs a comparing process for the detected voltage is provided in the controller 22, then the controller 22 can detect the driving voltage for driving the ultrasonic transducer 2a, perform the comparing process for the detected driving voltage, and detect a fluctuation in the driving voltage relative to a preset determination reference. Similarly to the instances related to the impedance or the driving power, the mechanical load exerted on the probe 2b can be thereby determined. In addition, the same functions and advantages as those of the first and the second embodiments can be exhibited.

A third embodiment of the present invention will be explained below. According to the first embodiment, the impedance when the ultrasonic transducer 2a is driven is detected, and the comparing process for the impedance is performed. According to the second embodiment, the driving power or the driving voltage for driving the ultrasonic transducer 2a is detected, and the comparing process is performed for the driving power or the driving voltage. According to the third embodiment, by contrast, the resonance frequency of the ultrasonic transducer 2a is detected, and a comparing process is performed for the resonance frequency.

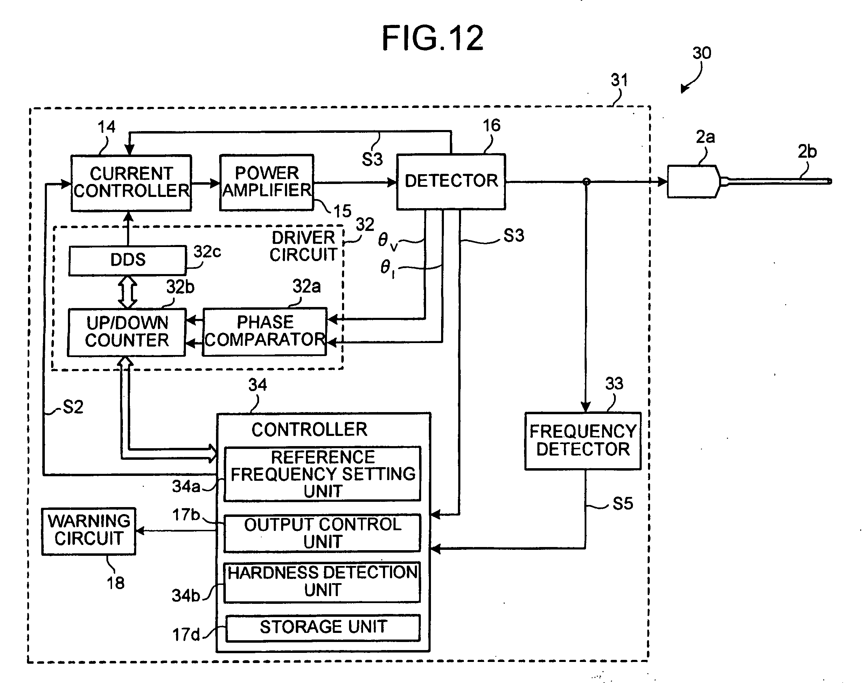

Fig. 12 is a block diagram which depicts an example of the basic configuration of an ultrasonic surgical system according to the third embodiment of the present invention. A control device 31 of this ultrasonic surgical system 30 includes a hardness detection unit 34b in place of the impedance processing unit 17c of the controller 17 arranged in the control device 1 of the ultrasonic surgical system 10 according to the first embodiment, and a reference frequency setting unit 34a in place of the resonance point detection unit 17a thereof. In addition, the control device 31 includes a frequency detector 33 in rear of the detector 16, and a digital driver circuit 32 in place of the analog driver circuit 13. The other constituent parts of the ultrasonic surgical system 30 are identical to those of the ultrasonic surgical system 10 according to the first embodiment, and like parts are designated with like reference signs.

The driver circuit 32 is realized by a digital phase synchronization circuit composed of a phase comparator 32a, an UP/DOWN counter 32b, and a DDS 32c. The phase comparator 32a detects a phase difference between a current and a voltage of a driving signal based on a voltage phase signal $\theta_V$ and a current phase signal $\theta_I$ fed back from the detector 16. The phase comparator 32a generates a frequency control signal for controlling rise and fall of a frequency input from the controller 34 based on the detected phase difference, and outputs the generated frequency control signal to the UP/DOWN counter 32b. The UP/DOWN counter 32b determines a frequency of the driving signal input to the ultrasonic transducer 2a based on the frequency control signal input from the phase comparator 32a and the reference frequency signal input from the controller 34, and outputs a frequency setting signal corresponding to the frequency to the DDS 32c. The DDS 32c outputs a sine wave of the frequency corresponding to the frequency setting signal input from the UP/DOWN counter 32b based on the frequency setting signal. The driver circuit 32 thereby outputs the driving signal with the reference frequency to the current controller 14 when the ultrasonic transducer 2a is activated, and then outputs the driving signal with the resonance frequency fr of the ultrasonic transducer 2a or a frequency near the resonance frequency fr to the current controller 14.

The driver circuit 32 may be realized by using the analog phase synchronization circuit. The driver circuit 32, however, is preferably realized by using the digital phase synchronization circuit. This is because if the analog phase synchronization circuit is used, frequency characteristics of the phase synchronization circuit change according to a temperature change or the like.

The frequency detector 33 receives the driving signal output from the detector 16, and detects the frequency of the received driving signal. If the ultrasonic transducer 2a is in a steady driving state, the frequency of the driving signal is a frequency output by a PLL control exercised by the driver circuit 32, and corresponds to the resonance frequency fr of the ultrasonic transducer 2a. Namely, the frequency detector 33 detects the resonance frequency fr of the ultrasonic transducer 2a. In this case, the frequency detector 33 outputs a frequency detection signal S5 corresponding to the detected resonance frequency fr to the controller 34. The frequency detector 33 may detect the frequency of the driving signal by receiving the driving signal which is power-amplified by the power amplifier 15, or by receiving the frequency setting signal output from the UP/DOWN counter 32b.

The controller 34 includes the reference frequency setting unit 34a, the hardness detection unit 34b, the output control unit 17b, and the storage unit 17b. The reference frequency setting unit 34a sets the reference frequency of the driving signal, and the controller 34 outputs the reference frequency signal corresponding to the reference frequency set by the reference frequency setting unit 34a to the driver circuit 32. The reference frequency setting unit 34a discriminates each time (the time ta to the time tc) required until the ultrasonic transducer 2a turns into the steady driving state from a time in which a hardness detecting process, to be explained later, is performed. The reference frequency setting unit 34a sets the reference frequency suited to the ultrasonic transducer 2a at each time. For example, the reference frequency setting unit 34a sets the resonance frequency fr stored in the storage unit 17d in advance as the reference frequency at the time ta to the time tc, and sets a predetermined frequency stored in the storage unit 17d in advance as the reference frequency at the time in which the hardness detecting process is performed.

If the controller 34 receives the frequency detection signal S5, the hardness detection unit 34b performs the hardness detecting process for detecting a hardness of an object in contact with the probe 2b based on the resonance frequency fr corresponding to the received frequency detection signal S5. The hardness detection unit 34b compares the obtained resonance frequency fr with a preset determination reference frequency, thereby performing the hardness detecting process. The controller 34a stores the obtained resonance frequency in the storage unit 17d as a part of driving information, and manages the resonance frequency fr as a comparison parameter to be compared with the determination reference parameter.

The determination reference frequency is set as the determination reference parameter for the detected resonance frequency fr in advance. Generally, the resonance frequency fr of the ultrasonic transducer 2a changes proportionally to a mechanical load exerted on the probe 2b. For example, if the resonance frequency of the ultrasonic transducer 2a is a frequency f0 while the mechanical load is not exerted on the probe 2b (when the probe 2b is in a non-contact state), and the mechanical load exerted on the probe 2b is heavy, the resonance frequency fr greatly changes from the frequency fr. If the mechanical load is light, the resonance frequency fr changes to a frequency near the frequency f0. Accordingly, if this determination reference frequency is set within a range from a frequency equal to or higher than a highest resonance frequency corresponding to the mechanical load exerted on the probe 2b caused by the contact of the probe 2b with the treatment target to a frequency equal to or lower than the lowest resonance frequency corresponding to the mechanical load which may cause damage to the probe 2b, the hardness detection unit 34b compares this determination reference frequency with the resonance frequency detected fro the driving signal using this principle. In this case, it is possible to determine whether the mechanical load which may cause damage to the probe 2b is exerted on the probe 2b.

Namely, the hardness detection unit 34b compares this determination reference frequency with the resonance frequency fr detected from the driving signal, determines a degree of the mechanical load exerted on the probe 2b, and thereby detects the hardness of the object in contact with the probe 2b. For example, if the resonance frequency detected from the driving signal is higher than the determination reference frequency, the hardness detection unit 34b detects that the hardness of the object in contact with the probe 2b is a hardness which may cause damage to the probe 2b. In this case, a frequency f1 is set as the determination reference frequency in advance, the controller 34 stores the frequency f1 in the storage unit 17d and manages the frequency f1 as determination reference information. The frequency f1 is preferably set at a frequency near the lowest reference frequency corresponding to the mechanical load which causes damage to the probe 2b within a range of setting the determination reference frequency. By so setting, the hardness detection unit 34 can ensure detecting the hardness of the object in contact with the probe 2b which may cause damage to the probe 2b.

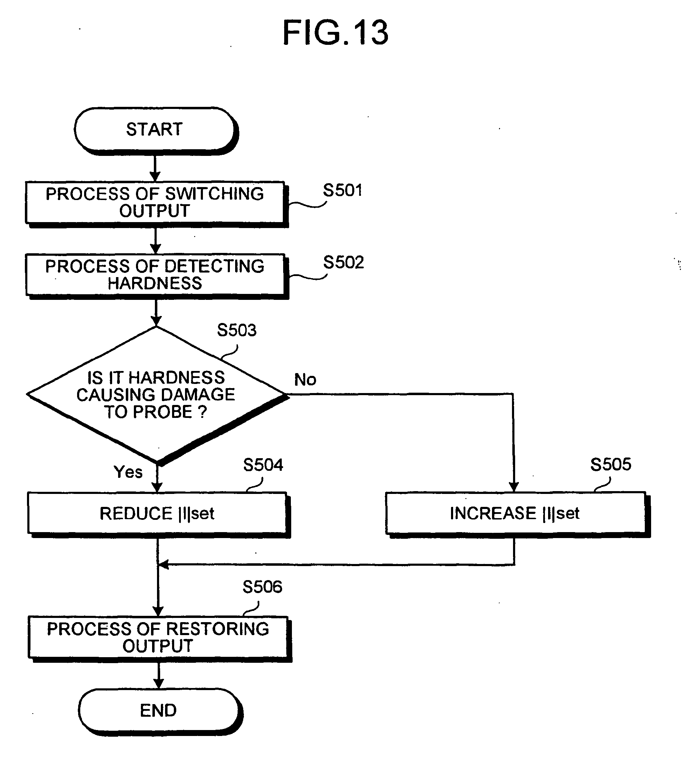

Fig. 13 is a flowchart of respective process procedures performed until the hardness detection unit 34b of the controller 34 detects the hardness of the object in contact with the probe 2b, and reduces the mechanical load exerted on the probe 2b or increases a reduced current of the driving signal according to the detected hardness. With reference to Fig. 13, if the hardness detection unit 34b is to detect the hardness of the object in contact with the probe 2b, the controller 34 switches an ultrasonic output of the ultrasonic transducer 2a from a medical treatment output to a hardness detecting output (at step S501). The ultrasonic output of the ultrasonic transducer 2a includes the medical treatment output for performing a medical treatment to the treatment target and the hardness detecting output for performing the hardness detecting process. The hardness detecting output is lower than the medical treatment output. Namely, by switching this ultrasonic output, the hardness detecting process can be performed safely and efficiently without excessively outputting the ultrasonic vibration to the object in contact with the probe 2b. The controller 34 controls the reference frequency setting unit 34a and the output control unit 17b to change a setting of the reference frequency and change the set current $|I|_{set}$, respectively, thereby performing the ultrasonic output switching process.

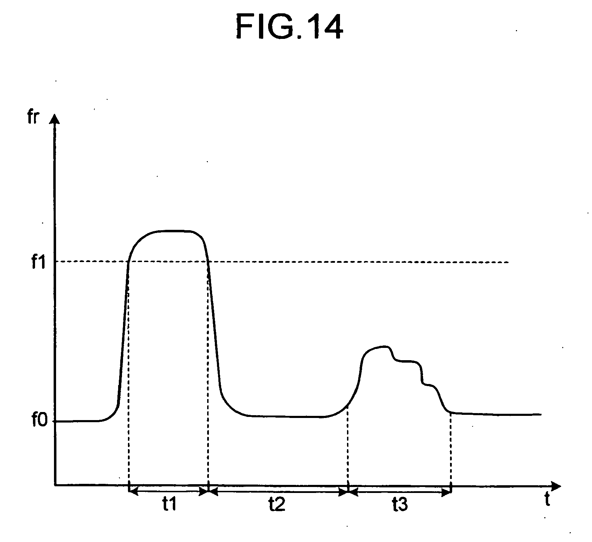

When the controller 34 receives the frequency detection signal S5 from the frequency detector 33 and reads the frequency f1 from the storage unit 17d as the determination reference frequency, the hardness detection unit 34b compares the resonance frequency fr corresponding to the received frequency detection signal S5 with the read frequency f1, and detects the hardness of the object in contact with the probe 2b based on a result of the comparing process (at step S502). Fig. 14 is a graph which specifically explains the result of the comparing process for comparing the resonance frequency fr with the frequency f1 performed by the hardness detection unit 34b. As shown in Fig. 14, if the frequency detector 33 detects the resonance frequency fr at the time t1, the hardness detection unit 34b compares the resonance frequency fr with the frequency f1, and determines that the resonance frequency fr is higher than the frequency f1. If the frequency detector 33 detects the resonance frequency fr at the time t2 and the time t3, the hardness detection unit 34b compares the resonance frequency fr with the frequency f1, and determines that the resonance frequency fr is equal to or lower than the frequency f1.

If the result of the comparing process between the resonance frequency fr and the frequency f1 indicates that the resonance frequency fr is higher than the frequency f1, the hardness detection unit 34b detects that the hardness of the object in contact with the probe 2b is the hardness which may cause damage to the probe 2b ("Yes" at step S503). In addition, the controller 34 controls the output control unit 17b to reduce the set current $|I|_{set}$ by as much as the current change amount $\Delta I_a$ similarly to the first embodiment. The output control unit 17b reduces the set current $|I|_{set}$ by as much as the current change amount $\Delta I_a$ under control of the controller 34 (at step S504), and outputs the current setting signal S2 corresponding to the reduced set current $|I|_{set}$ to the current controller 14. At step S504, the current $|I|$ of the driving signal is controlled to be lower than the set current $|I|_{set}$ corresponding to the set ultrasonic output set by the operator. It is thereby possible to reduce the amplitude of the ultrasonic vibration transmitted to the probe 2b, and reduce the mechanical load exerted on the probe 2b. Preferably, however, the process for reducing the set current $|I|_{set}$ at step S504 is performed until the resonance frequency fr is lower than the frequency f1.

If the result of the comparing process between the resonance frequency fr with the frequency f1 indicates that the resonance frequency fr is equal to or lower than the frequency f1, the hardness detection unit 34b detects that the hardness of the object in contact with the probe 2b is not the extent which may cause damage to the probe 2b ("No" at step S503). In addition, the controller 34 controls the output control unit 17b to increase the set current $|I|_{set}$ by as much as the current change amount $\Delta I_b$ similarly to the first embodiment. The output control unit 17b increases the set current

$|I|_{set}$ by as much as the current change amount $\Delta I_b$ under control of the controller 34 (at step S505), and outputs the current setting signal S2 corresponding to the increased set current $|I|_{set}$ to the current controller 14. In this case, the current $|I|$ of the driving signal is controlled to be increased up to the set current $|I|_{set}$ corresponding to the set ultrasonic output set by the operator. By so controlling, the ultrasonic transducer 2a can restore the amplitude of the ultrasonic vibration which has been once reduced to the original amplitude, and output the ultrasonic vibration which enables performing an appropriate medical treatment to the treatment target.

Thereafter, the controller 34 restores the ultrasonic output of the ultrasonic transducer 2a from the hardness detecting output to the medical treatment output (at step S506). If the set current $|I|_{set}$ is reduced at step S504, the controller 34 controls the reference frequency setting unit 34a and the output control unit 17b to return the changed setting of the reference frequency to the original setting, and to output the set current $|I|_{set}$ reduced at step S504, respectively. In this case, the current corresponding to the reduced set current $|I|_{set}$ is supplied to the ultrasonic transducer 2a, whereby the ultrasonic transducer 2a outputs the ultrasonic vibration the amplitude of which is reduced. If the set current $|I|_{set}$ is increased at step S504, the controller 34 controls the reference frequency setting unit 34a and the output control unit 17b to return the changed setting of the reference frequency to the original setting, and to output the set current $|I|_{set}$ increased at step S504, respectively. In this case, the current corresponding to the set current $|I|_{set}$, which is increased up to the set ultrasonic output set by the operator, is supplied to the ultrasonic transducer 2a, whereby the ultrasonic transducer 2a can efficiently outputs the ultrasonic vibration.

If a frequency f2 is set as the determination reference frequency in advance, and the hardness detection unit 34b is set to perform a comparing process for comparing the resonance frequency fr with the frequency f2 n times, then the hardness detection unit 34b can detect the hardness of the object in contact with the probe 2b in detail based on all results of the n comparing processes. Generally, when the probe 2b is in contact with the operation instrument, the heavy mechanical load is constantly exerted on the probe 2b and the fluctuation in the resonance frequency fr relative to the frequency fr is constantly large. In addition, when the probe 2b is in contact with calculus, the mechanical load exerted on the probe 2b fluctuates according to a state of the probe 2b in contact with the calculus, shapes of the calculus, or the like. The resonance frequency fr fluctuates relative to the frequency f0 similarly to the fluctuation in this mechanical load. Further, when the probe 2b is in contact with an object softer than the calculus such as the living tissue or the perfusion solution, or when the probe is out of contact with any object, the mechanical load exerted on the probe 2b is always light and the fluctuation in the resonance frequency fr relative to the frequency f0 is always small. Based on this principle, if the frequency f2 is set within the resonance frequency fr corresponding to the mechanical load exerted on the probe 2b which breaks the calculi, then the hardness detection unit 34b can determine that the object in contact with the probe 2b is either the hard object such as the operation instrument or the object, such as the living tissue or the perfusion solution, softer than the calculus, or determine that the probe is out of contact with an object.

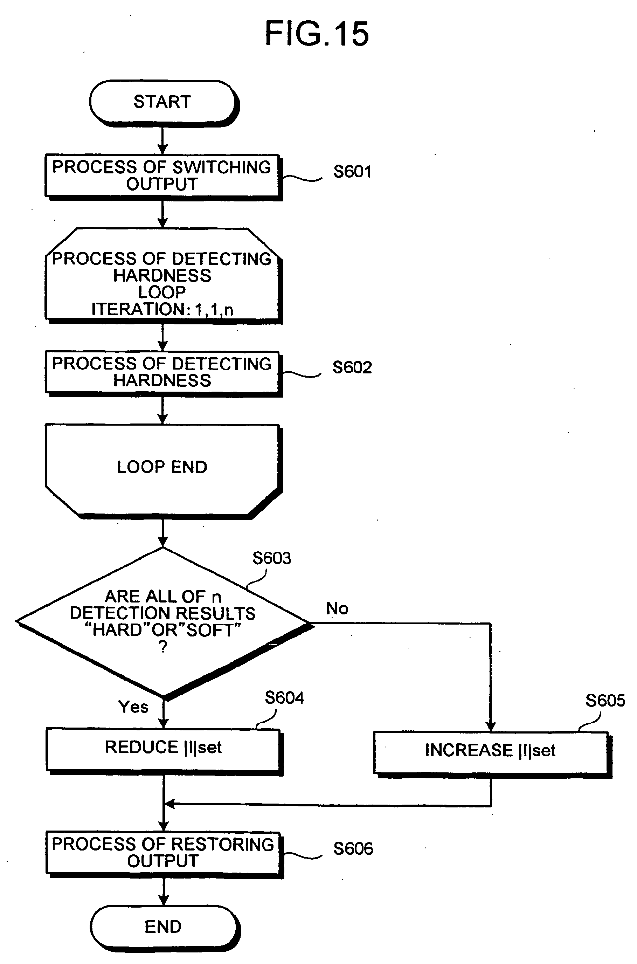

Fig. 15 is a flowchart of respective process procedures performed until the hardness detection unit 34b of the controller 34 performs the hardness detecting process for detecting the hardness of the object in contact with the probe 2b n times, and reduces the mechanical load exerted on the probe 2b or increases the reduced current of the driving signal according to the hardness detected based on all the result of the hardness detecting process. With reference to Fig. 15, if the hardness detection unit 34b is to detect the hardness of the object in contact with the probe 2b, the controller 34 switches the ultrasonic output of the ultrasonic transducer 2a from the medical treatment output to the hardness detecting output similarly to step S501 (at step S601).

When the controller 34 receives the frequency detection signal S5 from the frequency detector 33 and reads the frequency f2 from the storage unit 17d as the determination reference frequency, the hardness detection unit 34b performs the comparing process for comparing the resonance frequency fr corresponding to the received frequency detection signal S5 with the read frequency f2 n times, and detects the hardness of the object in contact with the probe 2b based on all results of the comparing processes (at step S602).

If the results of performing the comparing process between the resonance frequency fr and the frequency f2 the n times indicate that the resonance frequency fr is higher than the frequency f2 for all the n processes ("Yes" at step S603), then the hardness detection unit 34b detects that the hardness of the object in contact with the probe 2b is the hardness which may cause damage to the probe 2b, and that the object in contact with the probe 2b is the hard object such as the operation instrument. In this case, the controller 34 controls the output control unit 17b to reduce the set current $|I|_{set}$ by as much as the current change amount $\Delta I_a$ similarly to the first embodiment. The output control unit 17b reduces the set current $|I|set$ by as much as the current change amount $\Delta I_a$ under control of the controller 34 (at step S604), and outputs the current setting signal S2 corresponding to the reduced set current $|I|set$ to the current controller 14. At step S504, the current $|I|$ of the driving signal is controlled to be lower than the set current $|I|set$ corresponding to the set ultrasonic output set by the operator. It is thereby possible to reduce the amplitude of the ultrasonic vibration transmitted to the probe 2b, and reduce the mechanical load exerted on the probe 2b. Preferably, however, the process for reducing the set current $|I|_{set}$ at step S604 is performed until the resonance frequency fr is lower than the frequency f2.

If the results of performing the comparing process between the resonance frequency fr with the frequency f2 the n times indicate that the resonance frequency fr is lower than the frequency f2 for all the n processes ("Yes" at step S603), then the hardness detection unit 34b detects that the hardness of the object in contact with the probe 2b is not the extent which may cause damage to the probe 2b, and that the object in contact with the probe 2b is the soft object such as the living tissue or the perfusion solution softer than the calculus or detects that the probe 2b is out of contact with an object. In this case, the controller 34 controls the output control unit 17b to increase the set current $|I|_{set}$ by as much as the current change amount $\Delta I_b$ similarly to the first embodiment. The output control unit 17b increases the set current $|I|_{set}$ by as much as the current change amount $\Delta I_b$ under control of the controller 34 (at step S604), and outputs the current setting signal S2 corresponding to the increased set current $|I|_{set}$ to the current controller 14. In this case, the current $|I|$ of the driving signal is controlled to be increased up to the set current $|I|_{set}$ corresponding to the set ultrasonic output set by the operator. By so controlling, waste of the ultrasonic output by the ultrasonic transducer 2a can be suppressed, and damage to the living tissue due to the excessive ultrasonic output can be prevented. If the results of performing the comparing process between the resonance frequency fr and the frequency f1 the n times indicate that the resonance frequency fr is higher than the frequency f2 for the processes less than the n processes ("No" at step S603), then the hardness detection unit 34b detects that the hardness of the object in contact with the probe 2b is not the extent which may cause damage to the probe 2b, and that this object is the calculus. In this case, the controller 34 controls the output control unit 17b to increase the set current $|I|_{set}$, which is currently set, by as much as the current change amount $\Delta I_b$ up to the set current $|I|_{set}$ corresponding to the set ultrasonic output set by the operator. The output control unit 17b increases the set current $|I|_{set}$ by as much as the current change amount $\Delta I_b$ under control of the controller 34 (at step S605), and outputs the current setting signal S2 corresponding to the increased set current $|I|_{set}$ to the current controller 14. In this case, the current $|I|$ of the driving signal is controlled to be increased up to the set current $|I|_{set}$ corresponding to the set ultrasonic output set by the operator. The ultrasonic transducer 2a can thereby restore the amplitude of the ultrasonic vibration which has been once reduced to the original amplitude, and output the ultrasonic vibration which enables performing an appropriate medical treatment to the treatment target.

Thereafter, the controller 34 restores the ultrasonic output of the ultrasonic transducer 2a from the hardness detecting output to the medical treatment output similarly to step S506 (at step S606). If the set current $|I|_{set}$ is reduced at step S604, the current corresponding to the reduced set current $|I|_{set}$ is supplied to the ultrasonic transducer 2a, whereby the ultrasonic transducer 2a outputs the ultrasonic vibration the amplitude of which is reduced. If the set current $|I|_{set}$ is increased at step S604, the current corresponding to the set current $|I|_{set}$, which is increased up to the set ultrasonic output set by the operator, is supplied to the ultrasonic transducer 2a, whereby the ultrasonic transducer 2a can efficiently outputs the ultrasonic vibration.

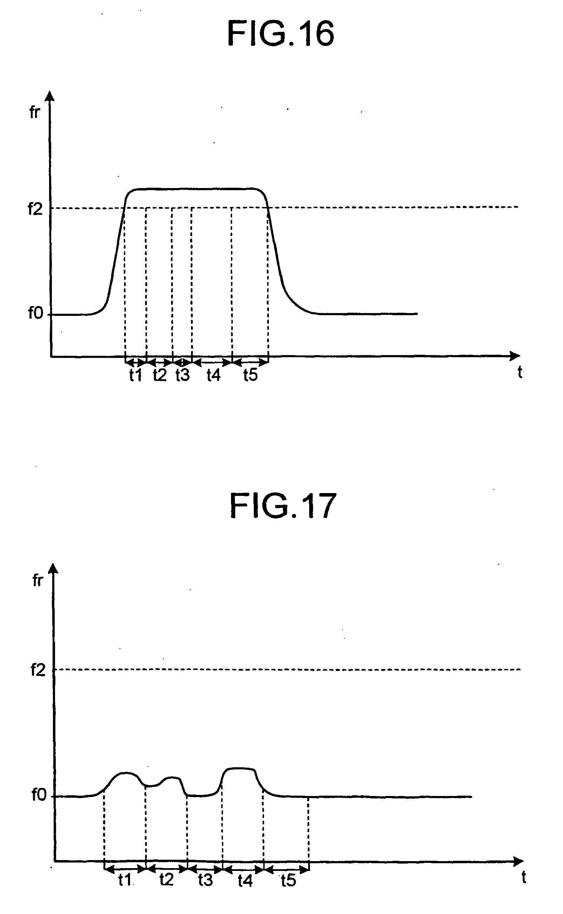

An instance in which the hardness detection unit 34b performs the hardness detecting process five times so as to detect the hardness of the object in contact with the probe 2b will now be explained specifically. Figs. 16 to 18 depict a first to a third examples in the resonance frequency fr of the ultrasonic transducer 2a relative to the time t, respectively. Namely, Figs. 16 to 18 are graphs for specifically explaining the result of performing the comparing process for comparing the resonance frequency fr with the frequency f2 by the hardness detection unit 34b the n times. The hardness detection unit 34b performs the comparing process between the resonance frequency fr and the frequency f2 once at each of a series of the time t1 to a time t5 of the time t shown in Figs. 16 to 18, and detects the hardness of the object in contact with the probe 2b based on the results of a total of five comparing processes.

If the resonance frequency fr fluctuates relative to the time t as shown in the first fluctuation example of Fig. 16, then the hardness detection unit 34 performs the comparing process between the resonance frequency fr and the frequency f2 the five times as explained above, and detects that the resonance frequency fr is higher than the frequency f2 for all the five processes. In this case, the hardness detection unit 34b can detect that the hardness of the object in contact with the probe 2b is the hardness which may cause damage to the probe 2b, and determine that this object is the hard object such as the operation instrument.

If the resonance frequency fr fluctuates relative to the time t as shown in the first fluctuation example of Fig. 17, then the hardness detection unit 34 performs the comparing process between the resonance frequency fr and the frequency f2 the five times as explained above, and detects that the resonance frequency fr is lower than the frequency f2 for all the five processes. In this case, the hardness detection unit 34b can detect that the hardness of the object in contact with the probe 2b is not the extent which may cause damage to the probe 2b, and determine that this object is the softer object such as the living tissue or the perfusion solution than the calculus.

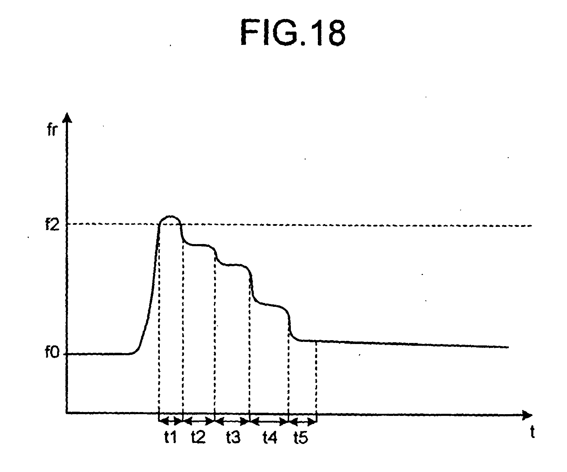

If the resonance frequency fr fluctuates relative to the time t as shown in the first fluctuation example of Fig. 18, then the hardness detection unit 34 performs the comparing process between the resonance frequency fr and the frequency f2 the five times as explained above, and detects that the resonance frequency fr is higher than the frequency f2 only for one of the five processes. In this case, the hardness detection unit 34b can detect that the hardness of the object in contact with the probe 2b is not the extent which may cause damage to the probe 2b, and determine that this object is the calculus.

The hardness detection unit 34b may perform this hardness detecting process while the medical treatment such as the lithotrity is performed, or at every predetermined timing set in advance. If the hard detection unit 34b performs the hard detecting process, the controller 34 may drive the ultrasonic transducer 2a at a constant voltage so that the ultrasonic transducer 2a outputs the ultrasonic vibration at the amplitude lower than that for the medical treatment output. The controller 34 may thereby switch the ultrasonic output from the medical treatment output to the hardness detecting output.

According to the third embodiment, the instance in which the amplitude of the ultrasonic vibration is not modulated has been explained. However, the present invention is not limited to the instance but can be applied to an instance in which the amplitude-modulated ultrasonic vibration is output.

According to the third embodiment, the resonance frequency of the ultrasonic transducer 2a is detected and the detected resonance frequency is compared with the preset determination reference frequency. The hardness of the object in contact with the probe is detected based on the result of the comparing process, and driving of the ultrasonic transducer is controlled according to the detected hardness. The amplitude of the ultrasonic vibration output to the probe 2b is thereby reduced or increased. Therefore, if the probe contacts with the hard object such as the operation instrument, it is possible to instantly detect that the hardness of the object in contact with the probe 2b is the hardness which may cause damage to the probe 2b, and reduce the mechanical load exerted on the probe 2b before the probe 2b is severely damaged. It is thereby possible to prevent damage to the probe 2b which may occur while the medical treatment on the treatment target is performed. Further, if the probe contacts with the object other than the hard object, it is possible to instantly detect that the hardness of the object in contact with the probe 2b is not the extent which may cause damage to the probe 2b, and efficiently output the ultrasonic vibration which enables performing the medical treatment to the treatment target. An operating efficiency of the medical treatment can be thereby improved.

Further, according to the third embodiment, the comparing process for comparing the detected resonance frequency with the preset determination reference frequency is performed a plurality of times, and the hardness of the object in contact with the probe 2b is detected based on all the results of the comparing processes. Therefore, it is possible to ensure detecting that the object in contact with the probe 2b is the hard object such as the operation instrument, the calculus, or the soft object such as the living tissue or the perfusion solution softer than the calculus. In addition, the driving of the ultrasonic transducer 2a is controlled according to the determined hardness of the object in contact with the probe 2b, and the amplitude of the ultrasonic vibration output to the probe 2b is thereby reduced or increased. Therefore, the mechanical load exerted on the probe 2b can be reduced before the probe 2b is severely damaged, and the ultrasonic vibration which enables the ultrasonic lithotrite or the like to perform the medical treatment can be efficiently output. The damage to the probe which may occur while the medical treatment is performed can be prevented, and the operating efficiency of the medical treatment can be improved.

A fourth embodiment of the present invention according to the present invention will be explained. According to the first to the third embodiments, the mechanical load exerted on the probe is reduced according to the result of the comparing process in relation to the driving information on the ultrasonic transducer. According to the fourth embodiment, wirings are provided on the probe, and the driving of the ultrasonic transducer is controlled to reduce the amplitude of the ultrasonic vibration when disconnection out of the wirings is detected.

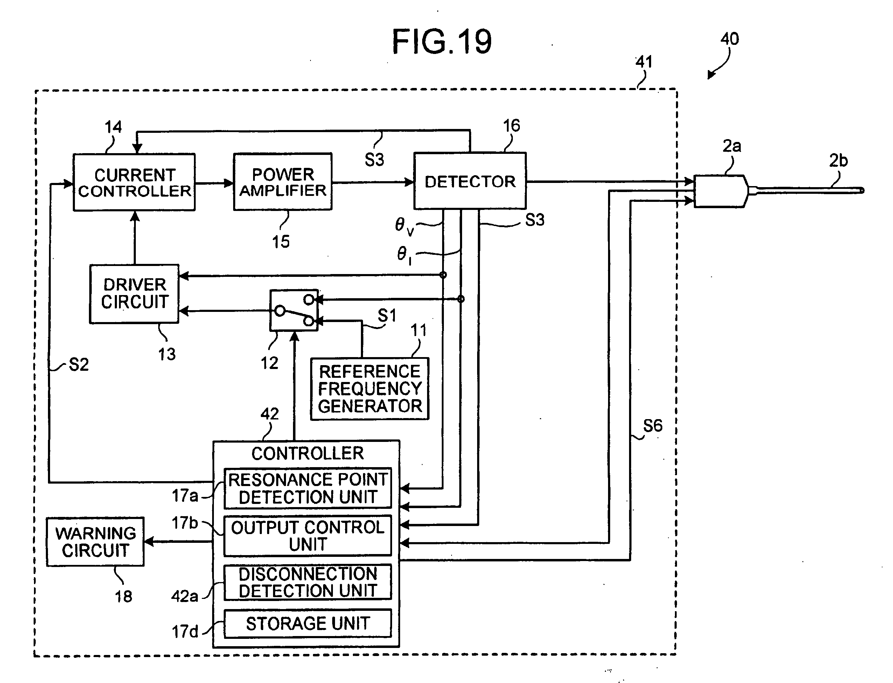

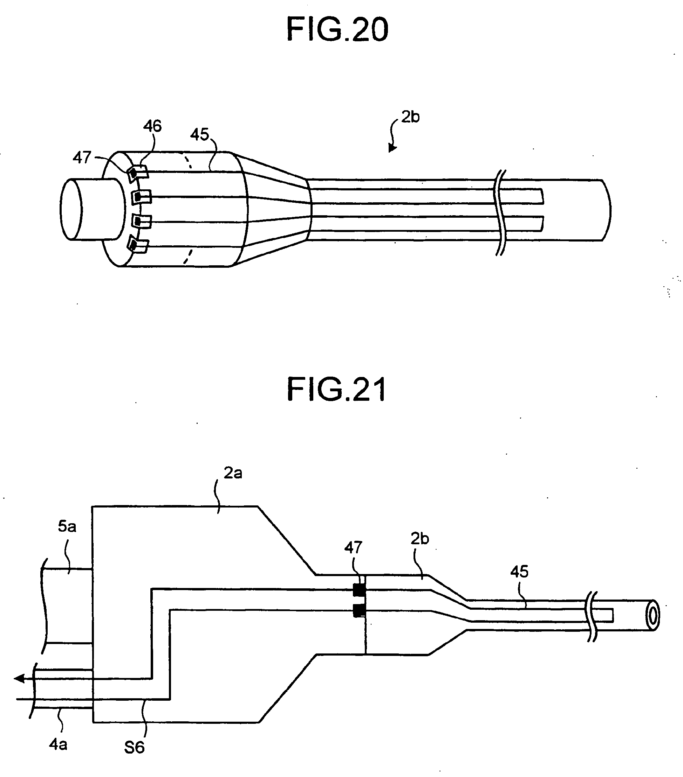

Fig. 19 is a block diagram which depicts an example of the basic configuration of a control device of an ultrasonic surgical system according to the fourth embodiment of the present invention. Fig. 20 is a schematic view which depicts an example of a state in which wirings are arranged on a probe of the ultrasonic surgical system according to the fourth embodiment of the present invention so as to be isolated from a probe. In Figs. 19 and 20, a control device 41 of an ultrasonic surgical system 40 includes a disconnection detection unit 42a in place of the impedance processing unit 17c of the controller 17 arranged in the control device 1 of the ultrasonic surgical system 10 according to the first embodiment. In addition, the disconnection detection unit 42a is electrically connected to a plurality of wirings 45 arranged on the probe 2b. The other constituent parts of the ultrasonic surgical system 40 are identical to those of the ultrasonic surgical system 10 according to the first embodiment, and like parts are designated with like reference signs.

When the control device 41 is turned on, the disconnection detection unit 42a causes a predetermined current to be constantly applied to the wirings 45 arranged on the probe 2b to thereby keep the wirings 45 continuous, and constantly detects a continuity impedance of the wirings 45 based on the current and a predetermined voltage applied to the wirings 45. The disconnection detection unit 42a compares the detected continuity impedance with a disconnection reference impedance to be explained later. If the continuity impedance is higher than the disconnection reference impedance, the disconnection detection unit 42a detects that one of the wirings 45 is disconnected.

Each wiring 45 is realized by covering a metal wire consisting of copper, iron, zinc, nickel, or the like or a combination thereof with an insulating film. As shown in Fig. 20, the wirings 45 are arranged on the probe 2b so as to reciprocate from a connection side on which the wirings 45 are connected to the ultrasonic transducer 2a toward a distal end of the probe 2b in contact with a treatment target on the probe 2b. A distance between the wirings 45 arranged on the probe 2b is preferably as small as possible. The wirings 45 are arranged on the probe 2b so as not to hinder various medical treatments using the ultrasonic vibration.

Two insulating sheets 46 are arranged on the probe 2b on the connection side with the ultrasonic transducer 2a for each wiring 45, and an electrode 47 is arranged on each insulating sheet 46. Each wiring 45 arranged on the probe 2b is electrically connected to the electrodes 47, whereby the two electrodes 47 are electrically connected to each other through one wiring 45 connected thereto. It is noted that the electrodes 47, the wirings 45, and the probe 2b are isolated from one another by coating films of the insulating sheets and the wirings 45.

Fig. 21 is an example of an electric connection state of the wiring 45 arranged on the probe 2b. In Fig. 21, one of the wirings 45 is shown. As shown in Fig. 21, the wiring 45, the electrodes 47, and the disconnection detection unit 42a of the controller 42 shown in Fig. 18 are electrically connected to one another through a wiring and a cable 4a which are arranged on the ultrasonic transducer 2a. When the disconnection detection unit 42 outputs a continuity signal S6 at a predetermined current and a predetermined voltage to the wiring 45, the continuity signal S6 is input to the wiring 45 through the cable 4a, the wiring on the ultrasonic transducer 2a, and one of the electrodes 47. The continuity signal S6 reaches the other electrode 47 through the wiring 45, and is input to the disconnection detection unit 42a through the wiring and the cable 41 which are provided on the ultrasonic transducer 2a. The disconnection detection unit 42a detects the continuity impedance of the wiring 45 based on the current and the voltage of the continuity signal S6 input through the wiring 45, and compares the preset disconnection reference impedance with the detected continuity impedance.

The controller 42 stores the disconnection reference impedance in the storage unit 17d as a determination reference for determining whether the wiring 45 is disconnected, and manages the disconnection reference impedance as determination reference information. The disconnection detection unit 42a compares the disconnection reference impedance read by the controller 42 with the detected continuity impedance. If the continuity impedance is higher than the disconnection reference impedance, the disconnection detection unit 42a detects the disconnection of the wiring 45.

The wiring 45 is disconnected when the operation instrument strongly contacts with the probe 2b and a high stress is applied to the wiring 45. Therefore, if the driving of the ultrasonic transducer 2a is controlled so as to reduce the amplitude of the ultrasonic vibration when the disconnection detection unit 42a detects the disconnection of the wiring 45, damage to the probe 2b can be prevented. In this case, similarly to the first embodiment, the controller 42 controls the output control unit 17b to reduce the set current $|I|$set by as much as the current change amount $\Delta Ia$, and outputs the current setting signal S2 corresponding to the reduced set current $|I|$set to the current controller 14. As a result, the driving of the ultrasonic transducer 2a is controlled so that the ultrasonic transducer 2a outputs the ultrasonic vibration the amplitude of which is reduced or the driving thereof is stopped. The mechanical load exerted on the probe 2b can be reduced, and damage to the probe 2b can be prevented.

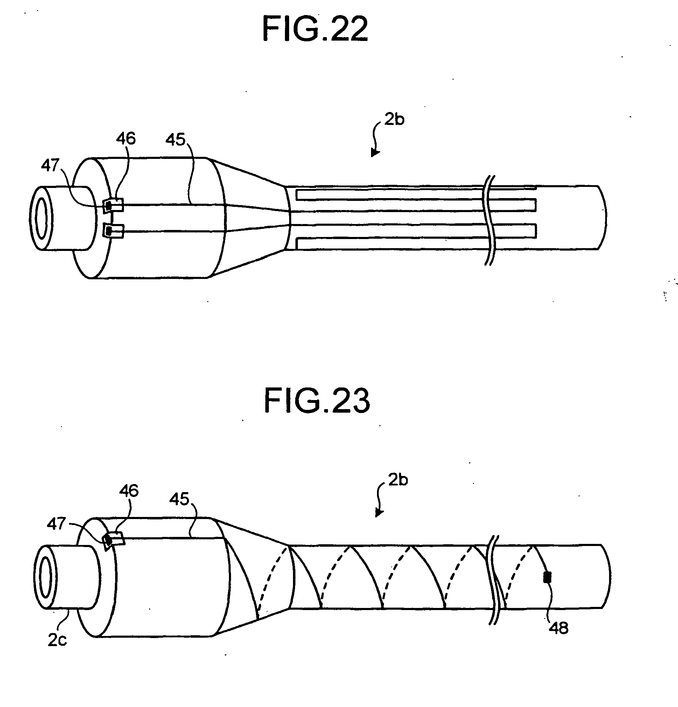

According to the fourth embodiment, a plurality of wirings 45 are arranged on the probe 2b as shown in Fig. 20. However, the number of wirings 45 is not limited to two or more. One wiring 45 may be provided on the probe 2b and arranged so as to reciprocate a plurality of times in a longitudinal direction of the probe 2b. Fig. 22 is a schematic view which depicts an example of an arrangement state in which one wiring 45 is arranged on the probe 2b isolated from the wiring 45 according to a first modification of the fourth embodiment. As shown in Fig. 22, both ends of the wiring 45 are electrically connected to the respective electrodes 47 isolated from the probe 2b by the insulating sheets 46, and the wiring 45 is arranged on the probe 2b so as to reciprocate in the longitudinal direction of the probe 2b a plurality of times. In this case, similarly to the fourth embodiment, the disconnection detection unit 42 can output and input the continuity signal S6. The first modification of the fourth embodiment can thus exhibit the same functions and advantages as those of the fourth embodiment.

According to the fourth embodiment and the first modification of the fourth embodiment, the instance in which the wiring 45 and the probe 2b are isolated from each other has been explained. However, the present invention is not limited to the arrangement state. Fig. 23 is a schematic view which depicts an example of an arrangement state when one wiring electrically connected to the probe 2b only by an electrode is arranged on the probe 2b. As shown in Fig. 23, an electrode 48 is arranged near the distal end of the probe 2b, one end of the wiring 45 is electrically connected to the electrode 47 on the insulating sheet 46, and the other end of the wiring 45 is electrically connected to the electrode 48. In addition, the wiring 45 is helically arranged on the probe 2b toward the longitudinal direction of the probe 2b.

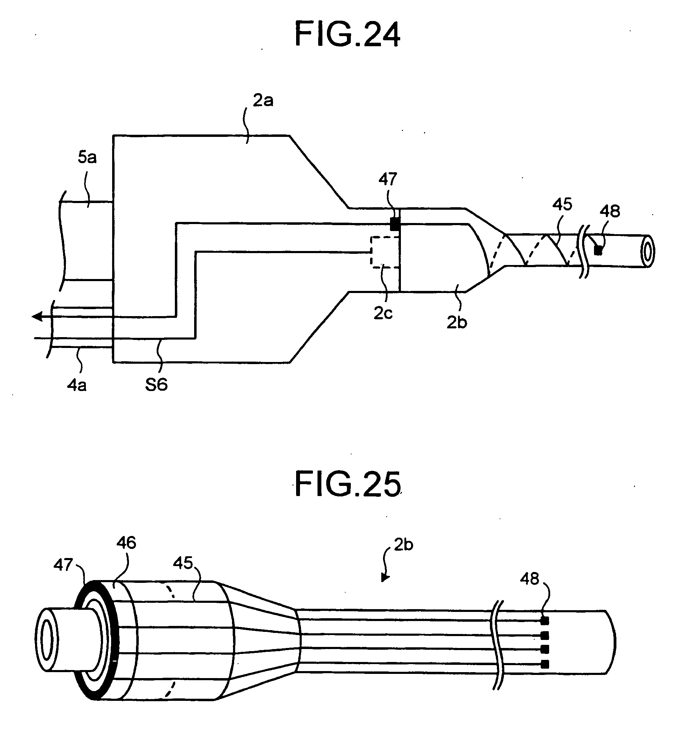

Fig. 24 is an example of an electrical connection state of the wiring 45 arranged on the probe 2b in an ultrasonic surgical system according to a second modification of the fourth embodiment. As shown in Fig. 24, the wiring 45, the electrodes 47 and 48, and the disconnection detection unit 42a of the controller 42 shown in Fig. 19 are electrically connected to one another through the wiring and the cable 4a which are arranged on the ultrasonic transducer 2a. The probe 2b includes a connection portion 2c detachably connected to the ultrasonic transducer 2a. The connection portion 2c is electrically connected to the wiring on the ultrasonic transducer 2a by connecting the probe 2b to the ultrasonic transducer 2a. In this case, the continuity signal S6 output from the disconnection detection unit 42a is output and input from and to the disconnection detection unit 42a through the cable 4a and the wiring on the ultrasonic transducer 2a, the probe 2b, the connection portion 2c, the wiring 45, and the electrodes 47 and 48. According to the second modification of the fourth embodiment, therefore, the disconnection detection unit 42a can output and input the continuity signal S6, and detect the disconnection of the wiring 45 similarly to the fourth embodiment and the first modification of the fourth embodiment. Thus, the second modification of the fourth embodiment exhibits the same functions and advantages as those of the fourth embodiment and the first modification of the fourth embodiment.

Further, according to the second modification of the fourth embodiment, one wiring 45 is helically arranged on the probe 2b as shown in Fig. 23. However, the present invention is not limited to this arrangement state. A plurality of wirings 45 may be helically arranged on the probe 2b, or a plurality of wirings 45 electrically connected to one electrode 47 may be arranged in parallel in the longitudinal direction of the probe 2b. Fig. 25 is a schematic view which depicts an example of an arrangement state when a plurality of wirings electrically connected to one electrode 47 are arranged in parallel in the longitudinal direction of the probe, and electrically connected to the probe through another electrode according to a third modification of the fourth embodiment. As shown in Fig. 25, one insulating sheet 46 is circumferentially arranged on the connection side of the probe 2b, and one electrode 47 is circumferentially arranged on this insulating sheet 46. One end of each wiring 45 is electrically connected to the electrode 47, and the other end thereof is electrically connected to the electrode 48. The wirings 45 are arranged in parallel in the longitudinal direction of the probe 2b. In this case, the connection portion 2c is electrically connected to the electrode 47 through the probe 2b, the electrode 48, and the wirings 45. The third modification of the fourth embodiment, therefore, exhibits the same functions and advantages as those of the fourth embodiment and the second modification of the fourth embodiment.

According to the fourth embodiment and the first to the third modifications of the fourth embodiment, one or a plurality of wirings covered with the insulating film are arranged on the probe. However, the present invention is not limited to the arrangement state. An insulating material may be printed on the probe in a desired arrangement state, and the wiring or wirings may be printed on the printed insulating material.

According to the fourth embodiment and the first to the third embodiments of the fourth embodiment, the wiring or wirings are arranged on the probe which transmits the ultrasonic vibration for performing various medical treatments to the treatment target. If the disconnection of one of the wirings is detected, the driving of the ultrasonic transducer 2a is controlled or stopped so as to reduce the amplitude of the ultrasonic vibration output to this probe 2b. Therefore, the mechanical load exerted on the probe 2b can be reduced before the probe 2b is damaged due to the contact of the probe 2b with the operation instrument. In addition, the ultrasonic surgical system which can prevent damage to the probe can be easily realized.

A fifth embodiment of the present invention will be explained. According to the first to the third embodiments, the driving of the ultrasonic transducer is controlled according to the mechanical load exerted on the probe, and the mechanical load is thereby reduced. In addition, according to the fourth embodiment, the driving of the ultrasonic transducer is controlled when the disconnection of the wiring is detected, and the mechanical load is thereby reduced. According to the fifth embodiment, the probe is covered with a protecting tool so as to physically protect the probe.

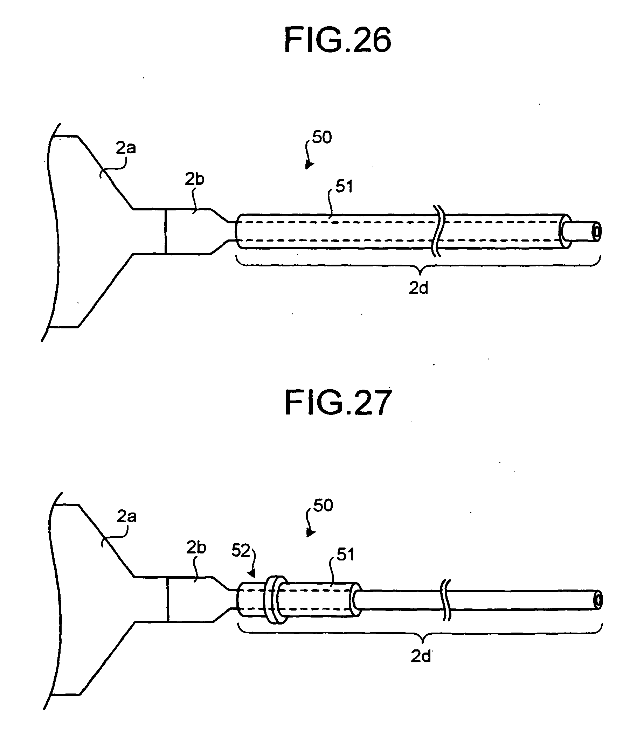

Fig. 26 is a schematic view which depicts an example of the protecting tool arranged on a probe of an ultrasonic surgical system according to the fifth embodiment of the present invention. In the probe 2b of an ultrasonic surgical system 50, a protecting tool 51 is arranged on an ultrasonic vibration transmitting unit 2d which transmits the ultrasonic vibration output from the ultrasonic transducer 2a to the treatment target. The other constituent parts of the ultrasonic surgical system 50 are identical to those of the ultrasonic surgical system 10 according to the first embodiment, and like parts are designated with like reference signs.

The protecting tool 51 consists of resin such as Teflon® or silicon, and is arranged on the probe 2b so as to cover the ultrasonic vibration transmitting unit 2d with the protecting tool 51. The protecting tool 51 covers the ultrasonic vibration transmitting unit 2d so as not to hinder various medical treatments performed by the ultrasonic surgical system 50. The protecting tool 51 is arranged on the probe 2b, for example, so as not to cover a distal end of the probe 2b which transmits the ultrasonic vibration to the treatment target and neighborhoods of the distal end.

The protecting tool 51 is of a sheet or cylindrical shape. If the sheet protecting tool 51 is arranged on the probe 2b, then the sheet protecting tool 51 is wound around the ultrasonic vibration transmitting unit 2d and the wound protecting tool 51 is fixedly attached to the probe 2b by an adhesive, a fusion treatment, or the like. If the cylindrical protecting tool 51 is arranged on the probe 2b, the cylindrical protecting tool 51 is detachably attached onto the probe 2b so that the ultrasonic transmitting unit 2d is inserted into the tool 51. In this case, the attached cylindrical protecting tool 51 is detachably attached onto the probe 2b by an elastic force of the protecting tool 51. Thus, the sheet or cylindrical protecting tool 51 can be arranged on the probe 2b without being detached from the probe due to the output of the ultrasonic vibration from the ultrasonic transducer 2a or the contact of the probe 2b with the rigid endoscope 7.

The protecting tool 51 preferably consists of heat-shrinkable resin. This is because when a heat treatment is carried out to the protecting tool 51 arranged on the probe 2b, this protecting tool 51 shrinks by heat and is attached to the probe 2b. An attachment strength of the protecting tool 51 on the probe 2b can be thereby intensified. Examples of this heat treatment include a method for outputting the ultrasonic vibration to the probe 2b covered with the protecting tool 51 for a short time, and heating the protecting tool 51 by friction between the protecting tool 51 and the probe 2b.

The probe 2b is then inserted into the rigid endoscope 7 as shown in Fig. 1, the probe to which the ultrasonic vibration is output is pressed against the treatment target, and the medical treatment can be thereby performed to this treatment target. However, the probe 2b may possibly be damaged due to the contact of the probe 2b with the rigid endoscope 7 while this medical treatment is being performed. For example, the probe 2 is often in contact with the rigid endoscope 7 at positions a to c shown in Fig. 1, particularly at the position a. The position a corresponds to a position near the insertion port 7c of the rigid endoscope 7, the position b corresponds to a distal end of the rigid endoscope 7, and the position c corresponds to a position near an intermediate part of a through port (not shown) of the rigid endoscope 7.

If the protecting tool 51 is arranged on the probe 2b as explained, the protecting tool 51 covers the ultrasonic vibration transmitting unit 2d of the probe 2b including the positions a to c. Therefore, the protecting tool 51 can prevent the probe 2b from directly contacting with the rigid endoscope 7, and prevent damage to the probe 2b caused by the contact of the probe 2b with the rigid endoscope 7. Further, since the protecting tool 51 is arranged on the probe 2b by the physical method as explained, the protecting tool 51 can be easily detached from the probe 2b by hands, a tool, or the like. Therefore, when the protecting tool 51 arranged on the probe 2b is damaged by the contact of the probe 2b with the rigid endoscope 7, the damaged protecting tool 51 can be easily replaced by a new protecting tool 51. The mechanical strength of the probe 2b can be thereby easily maintained.

According to the fifth embodiment, the protecting tool 51 covers the ultrasonic vibration transmitting unit 2d including the positions a to c, and thereby protects the probe 2b from the rigid endoscope 7. However, the present invention is not limited to the arrangement state. The protecting tool 51 may partially cover a desired position of the ultrasonic vibration transmitting unit 2d. Fig. 27 is a schematic view of the protecting tool 51 partially covering the ultrasonic vibration transmitting unit 2d of the probe 2b. As shown in Fig. 27, the protecting tool 51 partially covers the ultrasonic vibration transmitting unit 2d. In this case, the protecting tool 51 preferably covers the ultrasonic vibration transmitting unit 2d including the position a. By doing so, the protecting tool 51 can efficiently protect the probe 2b from the rigid endoscope 7, and damage to the probe 2b caused by the contact of the probe 2b with the rigid endoscope 7 can be efficiently prevented.

If the protecting tool 51 partially covers the ultrasonic vibration transmitting unit 2d, a position indicator 52 which indicates a position at which the ultrasonic vibration transmitting unit 2d is covered with the protecting tool 51 may be provided on the probe 2b. In this case, the protecting tool 51 is arranged based on the position indicator 52, thereby making it possible to ensure covering the desired position of the ultrasonic vibration transmitting unit 2d. The position indicator 52 may indicate the position at which the ultrasonic vibration transmitting unit 2d is covered with the protecting tool 51 and indicate the position of the probe 2b relative to the rigid endoscope 7.

According to the fifth embodiment, the ultrasonic vibration transmitting unit 2d of the probe 2b is covered with the protecting tool 51. Therefore, when the medical treatment is performed using the probe 2b inserted into the rigid endoscope 7, then the direct contact of the probe 2b with the rigid endoscope 7 can be inhibited and damage to the probe 2b caused by the contact of the probe 2b with the rigid endoscope 7 can be thereby easily prevented.

Further, this protecting tool 51 is provided on the probe 2b so as to be able to be easily detached from the probe 2b by hands, the tool, or the like. Therefore, the damaged protecting tool 51 can be easily replaced by a new protecting tool, and the mechanical strength of the probe 2b can be thereby easily maintained.

If the position indicator 52 which indicates the position at which the ultrasonic vibration transmitting unit 2d is covered with the protecting tool 52 is provided, this protection tool 51 is arranged based on the position indicator provided on the probe 2b. The probe 2b can be efficiently protected from the rigid endoscope 7, and damage to the probe 2b caused by the contact of the probe 2b with the rigid endoscope 7 can be efficiently prevented.

According to the first to the fifth embodiments, the instance of applying the present invention to the ultrasonic lithotrite which breaks the calculus in the hollow portion of the body and which sucks in broken particles of the calculus as one example of the ultrasonic surgical system and the probe has been explained below. However, the present invention is not limited to the instance. It can also be applied to a scissors type ultrasonic surgical system which coagulates and cuts the living tissue or the like, a hook type ultrasonic surgical system which peels off or cuts the living tissue or the like, and a suction type ultrasonic surgical system which emulsifies and sucks in the living tissue or the like, as well as various other ultrasonic surgical systems and probes such as an ultrasonic forceps.

According to the first and the second embodiments, the impedance of the ultrasonic transducer or the driving power for the ultrasonic transducer when the transducer is driven is detected based on the current and the voltage detected from the driving signal input to the ultrasonic transducer. However, the present invention is not limited to the embodiments. The impedance when the ultrasonic transducer is driven or the driving power may be detected based on the current corresponding to the current setting value set by the controller, and based on the voltage from the driving signal input to the ultrasonic transducer.

Although the invention has been described with respect to a specific embodiment for a complete and clear disclosure, the appended claims are not to be thus limited but are to be construed as embodying all modifications and alternative constructions that may occur to one skilled in the art which fairly fall within the basic teaching herein set forth.

**Claims**

**1.** An ultrasonic surgical system (10, 20, 30, 40) comprising:

an ultrasonic transducer (2a);

a probe (2b) connected to the ultrasonic transducer (2a) and which comes in contact with a treatment target;
a detector (16) which detects current and voltage which are supplied to the ultrasonic transducer (2a);
a driver (13) which drives the ultrasonic transducer to oscillate at a resonance point of the ultrasonic transducer;
a storage unit (17d) which stores a first parameter indicating a reference for determining whether a mechanical load exerted on the probe (2b) is a load which causes damage to the probe (2b); and
a controller (17, 22, 34, 42) which calculates a second parameter indicating the mechanical load exerted on the probe (2b) based on the voltage detected by the detector (16), and which outputs a signal for reducing the mechanical load to the driver (13) when the second parameter is higher than the first parameter.

2. The ultrasonic surgical system according to claim 1, wherein
the storage unit (17d) further stores a third parameter indicating a reference for determining whether an ultrasonic vibration for appropriately performing medical treatment to the treatment target is output, and
the controller (17, 22, 34, 42) outputs a signal for increasing an amplitude by vibration of the probe (2b) to the driver when the second parameter is lower than the third parameter.

3. The ultrasonic surgical system according to claim 1, wherein
the storage unit (17d) further stores a fourth parameter indicating a fluctuation range of the mechanical load, the fluctuation range indicating a contact state between a specific treatment target and the probe (2b), and
the controller (17, 22, 34, 42) calculates a fifth parameter corresponding to the mechanical load exerted on the probe (2b) based on a first current supplied to the ultrasonic transducer and a voltage corresponding to the first current, calculates a sixth parameter corresponding to the mechanical load exerted on the probe based on a second current lower than the first current and a voltage corresponding to the second current, and outputs a signal for reducing the mechanical load to the driver when the fifth parameter and the sixth parameter are higher than an upper limit indicated by the fourth parameter.

4. The ultrasonic surgical system according to claim 3, wherein the controller (17, 22, 34, 42) outputs a signal for increasing an amplitude by vibration of the probe when the fifth parameter and the sixth parameter are lower than a lower limit indicated by the fourth parameter.

5. The ultrasonic surgical system according to claim 1, wherein the first parameter and the second parameter indicate an impedance of the ultrasonic transducer.

6. The ultrasonic surgical system according to claim 1, wherein the first parameter and the second parameter indicate a power supplied to the ultrasonic transducer.

7. An ultrasonic surgical system (10, 20, 30, 40) comprising:

an ultrasonic transducer (2a);
a probe (2b) connected to the ultrasonic transducer and which comes in contact with a treatment target;
a detector (16) which detects a resonance frequency from a driving signal input to the ultrasonic transducer;
a driver (13) which drives the ultrasonic transducer to oscillate at a resonance point of the ultrasonic transducer;
a storage unit (17d) which stores a first reference parameter for determining whether a hardness of an object in contact with the probe is a hardness which causes damage to the probe; and
a controller (17, 22, 34, 42) which outputs a signal for reducing a mechanical load exerted on the probe to the driver (13) when the resonance frequency detected by the detector (16) is higher than the first reference parameter.

8. The ultrasonic surgical system according to claim 7, wherein
the storage unit (17d) stores a second reference parameter indicating a fluctuation range of the mechanical load, the fluctuation range indicating a contact state between a specific treatment target and the probe, and
the controller (17, 22, 34, 42) compares the resonance frequency fluctuating with the second reference parameter predetermined times, and outputs a signal for reducing the mechanical load when all of comparison results indicate that the resonance frequency exceeds the fluctuation range indicated by the second reference parameter.

**Patentansprüche**

1. Eine chirurgische Ultraschallvorrichtung (10, 20, 30, 40), die umfasst:

einen Ultraschallwandler (2a);

eine Sonde (2b), die mit dem Ultraschallwandler (2a) verbunden ist und mit einem zu behandelnden Ziel in Berührung kommt;

einen Detektor (16), der den Strom und die Spannung, die an den Ultraschallwandler (2a) angelegt werden, misst;

einen Treiber (13), der den Ultraschallwandler zum Schwingen am Resonanzpunkt des Ultraschallwandlers antreibt;

eine Speichereinheit (17d), die einen ersten Parameter speichert, der einen Referenz zur Bestimmung, ob eine auf die Sonde (2b) ausgeübte mechanische Kraft eine die Sonde (2b) zerstörende Kraft ist, angibt, und

eine Steuereinrichtung (17, 22, 34, 42), die einen zweiten Parameter auf der Grundlage der von dem Detektor (16) gemessenen Spannung berechnet, der die auf die Sonde (2b) ausgeübte Kraft angibt und der ein Signal zur Verminderung der mechanischen Kraft an den Treiber (13) abgibt, wenn der zweite Parameter höher als der erste Parameter ist.

2. Die chirurgische Ultraschallvorrichtung nach Anspruch 1, bei der

die Speichereinheit (17b) ferner einen dritten Parameter speichert, der eine Referenz zur Bestimmung angibt, ob eine Ultraschallschwingung zur geeigneten Ausführung einer medizinischen Behandlung an dem Behandlungsziel ausgegeben wird, und

die Speichervorrichtung (17, 22, 34, 42) ein Signal zur Erhöhung der Vibrationsamplitude der Sonde (2b) an den Treiber abgibt, wenn der zweite Parameter kleiner ist als der dritte Parameter.

3. Die chirurgische Ultraschallvorrichtung nach Anspruch 1, bei der

die Speichereinheit (17d) ferner einen vierten Parameter speichert, der einen Schwankungsbereich der mechanischen Kraft angibt, wobei der Schwankungsbereich einen Berührungszustand zwischen einem speziellen Behandlungsziel und der Sonde (2b) angibt, und

die Steuereinrichtung (17, 22, 34, 42) einen fünften Parameter auf der Grundlage eines ersten an den Ultraschallwandler angelegten Stroms und einer dem ersten Strom entsprechenden Spannung berechnet, die der auf die Sonde (2b) ausgeübten mechanischen Kraft entspricht; die einen sechsten Parameter auf der Grundlage eines zweiten Stroms, der geringer ist als der erste Strom, und einer Spannung, die dem zweiten Strom entspricht, berechnet, der der auf die Probe ausgeübten mechanischen Kraft entspricht, und ein Signal zur Verminderung der mechanischen Kraft an den Treiber abgibt, wenn der fünfte Parameter und der sechste Parameter größer sind als ein durch den vierten Parameter bestimmter oberer Grenzwert.

4. Die chirurgische Ultraschallvorrichtung nach Anspruch 3, bei der die Steuereinrichtung (17, 22, 34, 42) ein Signal zur Erhöhung der Vibrationsamplitude der Sonde abgibt, wenn der fünfte Parameter und der sechste Parameter niedriger sind als ein durch den vierten Parameter bestimmter Grenzwert.

5. Die chirurgische Ultraschallvorrichtung nach Anspruch 1, bei der der erste Parameter und der zweite Parameter die Impedanz des Ultraschallwandlers angeben.

6. Die chirurgische Ultraschallvorrichtung nach Anspruch 1, bei der der erste Parameter und der zweite Parameter die an den Ultraschallwandler abgegebene Leistung angeben.

7. Eine chirurgische Ultraschallvorrichtung (10, 20, 30, 40), die umfasst:

einen Ultraschallwandler (2a);

eine Sonde (2b), die mit dem Ultraschallwandler verbunden ist und mit einem Behandlungsziel in Berührung kommt;

eine Detektor (16), der aus dem Treibereingangssignal an den Ultraschallwandler eine Resonanzfrequenz misst;

ein Treiber (13), der den Ultraschallwandler mit einer Resonanzfrequenz des Ultraschallwandlers zu Schwingungen antreibt;

eine Speichereinheit (17d), die einen ersten Referenzparameter für die Bestimmung speichert, ob die Härte eines mit der Sonde in Berührung stehenden Objekts eine Härte ist, die zu einer Beschädigung der Sonde führt, und

eine Steuereinrichtung (17, 22, 34, 42), die ein Signal zur Verminderung einer auf die Sonde ausgeübten mechanischen Kraft an den Treiber (13) abgibt, wenn die von dem Detektor (16) festgestellte Resonanzfrequenz größer ist als der erste Referenzparameter.

8. Die chirurgische Ultraschallvorrichtung nach Anspruch 7, bei der

die Speichereinheit einen zweiten Referenzparameter speichert, der einen Schwankungsbereich der mechanischen Kraft angibt, wobei der Schwankungsbereich einen Berührungszustand zwischen einem speziellen Behandlungsziel und der Sonde angibt, und

die Steuereinrichtung (17, 22, 34, 42) die Resonanzfrequenz, die mit dem zweiten Referenzparameter über eine vorgegeben Zeit schwankt vergleicht, und ein Signal zur Verminderung der mechanischen Kraft abgibt wenn alle Vergleichsergebnisse anzeigen, das die Resonanzfrequenz den Schwankungsbereich überschreitet, der durch den zweiten Referenzparameter gegeben ist.

**Revendications**

1. Système chirurgical ultrasonique (10, 20, 30, 40) comprenant :

un transducteur ultrasonique (2a) ;
une sonde (2b) connectée au transducteur ultrasonique (2a) et qui vient en contact avec une cible de traitement ;
un détecteur (16) qui détecte un courant et une tension qui sont délivrés au transducteur ultrasonique (2a) ;
un pilote (13) qui commande le transducteur ultrasonique pour le faire osciller à un point de résonance du transducteur ultrasonique ;
une unité de stockage (17d) qui stocke un premier paramètre indiquant une référence pour déterminer si une charge mécanique exercée sur la sonde (2b) est une charge qui cause un dommage à la sonde (2b) ; et
un contrôleur (17, 22, 34, 42) qui calcule un deuxième paramètre indiquant la charge mécanique exercée sur la sonde (2b) sur la base de la tension détectée par le détecteur (16), et qui délivre en sortie un signal pour réduire la charge mécanique à destination du pilote (13) lorsque le deuxième paramètre est supérieur au premier paramètre.

2. Système chirurgical ultrasonique selon la revendication 1, dans lequel
l'unité de stockage (17d) stocke en outre un troisième paramètre indiquant une référence pour déterminer si une vibration ultrasonore pour exécuter de façon appropriée un traitement médical sur la cible de traitement est délivrée en sortie, et
le contrôleur (17, 22, 34, 42) délivre en sortie un signal pour augmenter une amplitude par vibration de la sonde (2b) à destination du pilote lorsque le deuxième paramètre est inférieur au troisième paramètre.

3. Système chirurgical ultrasonique selon la revendication 1, dans lequel
l'unité de stockage (17d) stocke en outre un quatrième paramètre indiquant une plage de fluctuation de la charge mécanique, la plage de fluctuation indiquant un état de contact entre une cible de traitement spécifique et la sonde (2b), et
le contrôleur (17, 22, 34, 42) calcule un cinquième paramètre correspondant à la charge mécanique exercée sur la sonde (2b) sur la base d'un premier courant délivré au transducteur ultrasonique et d'une tension correspondant au premier courant, calcule un sixième paramètre correspondant à la charge mécanique exercée sur la sonde sur la base d'un deuxième courant inférieur au premier courant et d'une tension correspondant au deuxième courant, et délivre en sortie un signal pour réduire la charge mécanique à destination du pilote lorsque le cinquième paramètre et le sixième paramètre sont supérieurs à une limite supérieure indiquée par le quatrième paramètre.

4. Système chirurgical ultrasonique selon la revendication 3, dans lequel le contrôleur (17, 22, 34, 42) délivre en sortie un signal pour augmenter une amplitude par vibration de la sonde lorsque le cinquième paramètre et le sixième paramètre sont inférieurs à une limite inférieure indiquée par le quatrième paramètre.

5. Système chirurgical ultrasonique selon la revendication 1, dans lequel le premier paramètre et le deuxième paramètre indiquent une impédance du transducteur ultrasonique.

6. Système chirurgical ultrasonique selon la revendication 1, dans lequel le premier paramètre et le deuxième paramètre indiquent une puissance fournie au transducteur ultrasonique.

7. Système chirurgical ultrasonique (10, 20, 30, 40) comprenant :

un transducteur ultrasonique (2a) ;
une sonde (2b) connectée au transducteur ultrasonique et qui vient en contact avec une cible de traitement ;
un détecteur (16) qui détecte une fréquence de résonance à partir d'un signal pilote entré dans le transducteur ultrasonique ;

un pilote (13) qui commande le transducteur ultrasonique pour le faire osciller à un point de résonance du transducteur ultrasonique ;

une unité de stockage (17d) qui stocke un premier paramètre de référence pour déterminer si une dureté d'un objet en contact avec la sonde est une dureté qui cause un dommage à la sonde ; et

un contrôleur (17, 22, 34, 42) qui délivre en sortie un signal pour réduire une charge mécanique exercée sur la sonde à destination du pilote (13) lorsque la fréquence de résonance détectée par le détecteur (16) est supérieure au premier paramètre de référence.

8. Système chirurgical ultrasonique selon la revendication 7, dans lequel

l'unité de stockage (17d) stocke un deuxième paramètre de référence indiquant une plage de fluctuation de la charge mécanique, la plage de fluctuation indiquant un état de contact entre une cible de traitement spécifique et la sonde, et le contrôleur (17, 22, 34, 42) compare la fréquence de résonance fluctuante avec le deuxième paramètre de référence un nombre prédéterminé de fois, et délivre en sortie un signal pour réduire la charge mécanique lorsque tous les résultats de comparaison indiquent que la fréquence de résonance excède la plage de fluctuation indiquée par le deuxième paramètre de référence.

# FIG.1

EP 1 495 727 B1

# FIG.2

EP 1 495 727 B1

# FIG.3

# FIG.4

# FIG.5

```
                          START

                   PROCESS OF DETECTING        S101
                       IMPEDANCE |Z|

                   PROCESS OF COMPARING        S102
                       CURRENT VALUE

                                               S103
         Yes
                          |I| < Ith?
                                    No

  PROCESS OF              S104
  COMPARING LOUER
  IMPEDANCE LIMIT

                S105
                          No
      |Z| < R2?
                                    PROCESS OF UPPER       S106
     Yes                            IMPEDANCE LIMIT

                                                    S107
                                                         No
                                        |Z| > R1?
               S109
                                       Yes

  INCREASE |I|set                   REDUCE |I|set         S108
```

# FIG.6

# FIG.7

```
                    ┌──────────────┐
                    │    START     │
                    └──────┬───────┘
                           │
         ┌─────────────────▼──────────────┐
         │  PROCESS OF DETECTING          │  S201
         │  IMPEDANCE |Z|                 │
         └──────────────┬─────────────────┘
                        │
                   ◇ S202
              |I|set UPON              I_L
              DETELTING CURRENT
              VALVE ?
                   │ I_H
```

PROCESS OF DETECTING IMPEDANCE |Z|  S201

|I|set UPON DETELTING CURRENT VALVE ?  S202

STORE |Z| AS $|Z|_H$  S203

STORE |Z| AS $|Z|_L$  S204

PROCESS OF COMPARING IMPEDANCE  S205

$|Z|_H > R3$ AND $|Z|_L > R3$?  S206

No

Yes

$|Z|_H \leqq R3$ AND $|Z|_L \leqq R3$?  S207

No

Yes

INCREASE |I|set  S208

REDUCE |I|set  S209

FIG.8

# FIG.9

# FIG.10

```
                              ┌─────────────┐
                              │    START    │
                              └──────┬──────┘
                                     │
                         ┌───────────▼───────────┐
                         │ PROCESS OF DETECTING  │ S301
                         │      POWER |W|        │
                         └───────────┬───────────┘
                                     │
                         ┌───────────▼───────────┐
                         │ PROCESS OF COMPARING  │ S302
                         │     CURRENT VALUE     │
                         └───────────┬───────────┘
                                     │           S303
                    Yes         ◇────▼────◇
            ┌─────────────────  │  |I| < Ith?  │
            │                   ◇─────────◇
            │                        │ No
 ┌──────────▼──────────┐             │
 │ PROCESS OF COMPARING │ S304        │
 │  LOWER POWER LIMIT   │             │
 └──────────┬──────────┘             │
            │    S305                 │
        ◇───▼───◇        No           │
        │ |W| < W2? ├──────────────┐  │
        ◇───────◇                  │  │
            │ Yes         ┌────────▼──▼────────┐
            │             │ PROCESS OF COMPARING │ S306
            │             │  UPPER POWER LIMIT   │
            │             └─────────┬──────────┘
            │                       │      S307
            │                   ◇───▼───◇       No
   S309     │                   │ |W| > W1? ├──────────►
 ┌──────────▼──────────┐        ◇───────◇
 │   INCREASE |I|set   │            │ Yes
 └──────────┬──────────┘   ┌────────▼────────┐ S308
            │              │  REDUCE |I|set  │
            │              └────────┬────────┘
            └───────────────────────┘
```

# FIG.11

```
                    START

                       ↓
        PROCESS OF DETECTING        S401
           POWER |W|

                       ↓
                                    S402
            |I|set UPON                      I_L
        DETECTING CURRENT  ──────────────────────┐
            VALUE ?                              │
                                                 │
              I_H ↓          S403                │      S404
        STORAGE |W| AS |W|_H          STORAGE |W| AS |W|_L
                                                 │
                       ↓←───────────────────────┘
        PROCESS OF COMPARING         S405
             POWER

                       ↓
                             S406
           |W|_H > W3                No
              AND        ──────────────────┐
           |W|_L > W3?                      │
                                            ↓        S407
             Yes                     |W|_H ≦ W3        No
                                        AND      ──────────┐
                                     |W|_L ≦ W3?           │
                                                           │
              ↓          S408          Yes ↓    S409       │
        INCREASE |I|set          REDUCE |I|set             │
              │                        │                   │
              └────────────────────────┴───────────────────┘
```

# FIG.12

EP 1 495 727 B1

# FIG.13

START

PROCESS OF SWITCHING OUTPUT — S501

PROCESS OF DETECTING HARDNESS — S502

S503

IS IT HARDNESS CAUSING DAMAGE TO PROBE ?

No

Yes

REDUCE |I|set — S504

INCREASE |I|set — S505

PROCESS OF RESTORING OUTPUT — S506

END

# FIG.14

# FIG.15

```
                    START

                      │
                      ▼
        ┌──────────────────────────┐
        │   PROCESS OF SWITCHING   │────  S601
        │         OUTPUT           │
        └──────────────────────────┘
                      │
                      ▼
        ┌──────────────────────────┐
        │  PROCESS OF DETECTING    │
        │        HARDNESS          │
        │          LOOP            │
        │   ITERATION: 1,1,n       │
        └──────────────────────────┘
                      │
                      ▼
        ┌──────────────────────────┐
        │  PROCESS OF DETECTING    │────  S602
        │        HARDNESS          │
        └──────────────────────────┘
                      │
                      ▼
        ┌──────────────────────────┐
        │        LOOP END          │
        └──────────────────────────┘
                      │
                      ▼              S603
              ◇ ARE ALL OF n ◇
           ◇ DETECTION RESULTS ◇────── No ──────┐
            ◇ "HARD"OR"SOFT" ◇                  │
                  ◇ ? ◇                         │
                    │                           │
                   Yes          S604            │          S605
                    ▼                           ▼
        ┌──────────────────────────┐   ┌──────────────────────────┐
        │      REDUCE |I|set       │   │     INCREASE |I|set      │
        └──────────────────────────┘   └──────────────────────────┘
                    │                           │
                    ◄───────────────────────────┘
                    ▼
        ┌──────────────────────────┐
        │  PROCESS OF RESTORING    │────  S606
        │         OUTPUT           │
        └──────────────────────────┘
                    │
                    ▼
                   END
```

# FIG.16

# FIG.17

# FIG.18

FIG.19

# FIG.20

# FIG.21

## FIG.22

## FIG.23

# FIG.24

# FIG.25

# FIG.26

# FIG.27

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 4979952 A
- US 5026387 A
- US 5425704 A
- US 5279547 A